(19) Europäisches Patentamt
European Patent Office
Office européen des brevets

(11) Veröffentlichungsnummer: **0 329 015 B1**

# (12) EUROPÄISCHE PATENTSCHRIFT

(45) Veröffentlichungstag der Patentschrift: **30.03.94**

(51) Int. Cl.[5]: **C07D 249/08**, A01N 43/653

(21) Anmeldenummer: **89102219.6**

(22) Anmeldetag: **09.02.89**

Die Akte enthält technische Angaben, die nach dem Eingang der Anmeldung eingereicht wurden und die nicht in dieser Patentschrift enthalten sind.

## (54) Substituierte Triazole.

(30) Priorität: **18.02.88 DE 3804981**

(43) Veröffentlichungstag der Anmeldung:
**23.08.89 Patentblatt 89/34**

(45) Bekanntmachung des Hinweises auf die Patenterteilung:
**30.03.94 Patentblatt 94/13**

(84) Benannte Vertragsstaaten:
**BE CH DE FR GB IT LI NL**

(56) Entgegenhaltungen:
**EP-A- 0 040 345**
**EP-A- 0 057 864**
**EP-A- 0 083 750**
**EP-A- 0 094 572**
**EP-A- 0 129 798**
**US-A- 4 549 900**

(73) Patentinhaber: **BAYER AG**

**D-51368 Leverkusen(DE)**

(72) Erfinder: **Kleefeld, Gerd, Dr.**
**Otto-Hahn-Strasse 87**
**D-4000 Düsseldorf 13(DE)**
Erfinder: **Dutzmann, Stefan, Dr.**
**Leinenweberweg 33**
**D-4000 Düsseldorf 13(DE)**

**EP 0 329 015 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue substituierte Triazole, mehrere Verfahren zu ihrer Herstellung sowie ihre Verwendung als Fungizide.

Es ist bereits bekannt, daß bestimmte substituierte Triazole eine fungizide Wirksamkeit besitzen (vgl. DE-A 2 431 407). So lassen sich zum Beispiel 1-(4-Chlorphenyl)-2-(1,2,4-triazol-1-yl)-propan-1-ol und 1-(4-Chlorphenyl)-2-(1,2,4-triazol-1-yl)-propan-1-on zur Bekämpfung von Pilzen verwenden. Die Wirksamkeit dieser Stoffe ist jedoch insbesondere bei niedrigen Aufwandmengen und Konzentrationen nicht in allen Anwendungsgebieten völlig zufriedenstellend.

Weiterhin ist bekannt, daß zahlreiche Triazolyl-Derivate, die als Brückenglied zwischen Azolyl-Rest und Carbonyl- bzw. Carbinol-Einheit eine unsubstituierte $CH_2$-Gruppe enthalten, fungizide Eigenschaften besitzen (vgl. EP-A 0 040 345, EP-A 0 057 864 und EP-A 0 094 572). Entsprechende Verbindungen mit einer

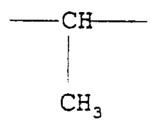

Gruppe an der betreffenden Stelle des Moleküls werden aber nicht beschrieben.

Schließlich geht aus der EP-A 0 083 750, der EP-A 0 129 798 und der US-A 4 549 900 hervor, daß auch Triazolyl-Verbindungen, in denen das Brückenglied zwischen Carbonyl- bzw. Carbinol-Funktion und Aryl-Rest ein disubstituiertes Kohlenstoffatom oder eine geminal disubstituierte Kohlenstoffkette darstellt, fungizid wirksam sind. Es werden aber keine Stoffe dieses Typs offenbart, in denen eine nur einfach substituierte, gesättigte oder ungesättigte Kohlenstoffkette mit zwei Kohlenstoffatomen die betreffenden Teile des Moleküls verbindet.

Es wurden nun neue substituierte Triazole der Formel

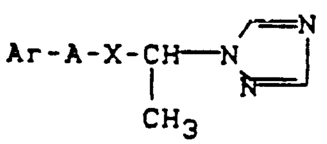 (I)

in welcher

Ar für Aryl mit 6 bis 10 Kohlenstoffatomen steht, das einfach oder mehrfach, gleichartig oder verschieden substituiert sein kann durch Halogen, Cyano, Nitro, jeweils geradkettiges oder verzweigtes Alkyl, Alkoxy und Alkylthio mit jeweils 1 bis 4 Kohlenstoffatomen, jeweils geradkettiges oder verzweigtes Halogenalkyl, Halogenalkoxy und Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl und Alkoximinoalkyl mit jeweils 1 bis 4 Kohlenstoffatomen im Alkoxyteil und 1 bis 4 Kohlenstoffatomen im Alkylteil, gegebenenfalls einfach oder mehrfach, gleichartig oder verschieden durch Halogen und/oder geradkettiges oder verzweigtes Alkyl mit 1 bis 4 Kohlenstoffatomen substituiertes Phenyl, gegebenenfalls einfach oder mehrfach, gleichartig oder verschieden durch Halogen und/oder geradkettiges oder verzweigtes Alkyl mit 1 bis 4 Kohlenstoffatomen substituiertes Phenoxy und/oder durch gegebenenfalls einfach oder mehrfach, gleichartig oder verschieden durch Halogen und/oder geradkettiges oder verzweigtes Alkyl mit 1 bis 4 Kohlenstoffatomen substituiertes Benzyloxy,

A für die Gruppe

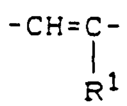 oder 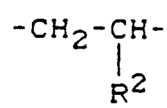

steht und

X für die Gruppen

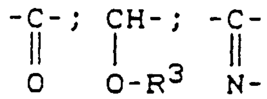 oder

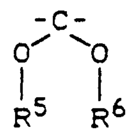

steht,

wobei

$R^1$ für Wasserstoff oder für geradkettiges oder verzweigtes Alkyl mit 1 bis 4 Kohlenstoffatomen steht,

$R^2$ für Wasserstoff oder für geradkettiges oder verzweigtes Alkyl mit 1 bis 4 Kohlenstoffatomen steht,

$R^3$ Wasserstoff, für jeweils geradkettiges oder verzweigtes Alkyl mit 1 bis 8 Kohlenstoffatomen, Alkenyl mit 3 bis 8 Kohlenstoffatomen oder Alkanoyl mit 1 bis 6 Kohlenstoffatomen im Alkanteil steht, außerdem für Cycloalkyl mit 3 bis 7 Kohlenstoffatomen steht, das einfach oder mehrfach, gleichartig oder verschieden substituiert sein kann durch Halogen, geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen, oder für Aralkyl mit 6 bis 10 Kohlenstoffatomen im Arylteil und 1 bis 4 Kohlenstoffatomen im geradkettigen oder verzweigten Alkylteil steht, wobei jeder der Reste einfach oder mehrfach, gleichartig oder verschieden im Arylteil substituiert sein kann durch Halogen, Cyano, Nitro, jeweils geradkettiges oder verzweigtes Alkyl, Alkoxy oder Alkylthio mit jeweils 1 bis 4 Kohlenstoffatomen und/oder jeweils geradkettiges oder verzweigtes Halogenalkyl, Halogenalkoxy
oder Halogenalkylthio mit jeweils 1 bis 4 Kohlenstoffatomen und 1 bis 9 gleichen oder verschiedenen Halogenatomen, oder für Aroyl mit 6 bis 10 Kohlenstoffatomen im Arylteil steht, wobei jeder dieser Arylreste einfach oder mehrfach, gleichartig oder verschieden substituiert sein kann durch Halogen, Cyano, Nitro, jeweils geradkettiges oder verzweigtes Alkyl, Alkoxy oder Alkylthio mit jeweils 1 bis 4 Kohlenstoffatomen und/oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen,

$R^4$ für Wasserstoff, geradkettiges oder verzweigtes Alkyl mit 1 bis 6 Kohlenstoffatomen oder für Cycloalkyl mit 3 bis 7 Kohlenstoffatomen steht, wobei jeder der Cycloalkylreste einfach oder mehrfach, gleichartig oder verschieden substituiert sein kann durch Halogen, geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen, oder für Aralkyl mit 6 bis 10 Kohlenstoffatomen im Arylteil und 1 bis 4 Kohlenstoffatomen im geradkettigen oder verzweigten Alkylteil steht, wobei jeder der Arylreste einfach oder mehrfach, gleichartig oder verschieden substituiert sein kann durch
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy und/oder Halogenalkylthio mit jeweisl 1 bis 4 Kohlenstoffatomen und 2 bis 9 gleichen oder verschiedenen Halogenatomen, oder für Aryl mit 6 bis 10 Kohlenstoffatomen steht, wobei jeder der Arylreste einfach oder mehrfach, gleichartig oder verschieden substituiert sein kann durch Halogen, Cyano, Nitro, jeweils geradkettiges oder verzweigtes Alkyl, Alkoxy oder Alkylthio mit jeweils 1 bis 4 Kohlenstoffatomen, jeweils

geradkettiges oder verzweigtes Halogenalkyl, Halogenalkoxy und/oder Halogenalkylthio mit jeweils 1 bis 4 Kohlenstoffatomen und 1 bis 9 gleichen oder verschiedenen Halogenatomen und

$R^5$ und $R^6$ unabhängig voneinander für Alkyl mit 1 bis 6 Kohlenstoffatomen oder Aralkyl mit 6 bis 10 Kohlenstoffatomen im Arylteil und 1 bis 4 Kohlenstoffatomen im geradkettigen oder verzweigten Alkylteil stehen, wobei jeder der Arylreste einfach oder mehrfach, gleichartig oder verschieden substituiert sein kann durch Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy und/oder Halogenalkylthio mit jeweils 1 bis 4 Kohlenstoffatomen und 1 bis 9 gleichen oder verschiedenen Halogenatomen, oder

$R^5$ und $R^6$ gemeinsam für einen Alkylenrest mit 2 bis 4 Kohlenstoffatomen stehen, wobei der Alkylenrest einfach oder mehrfach, gleichartig oder verschieden substituiert sein kann durch geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, Alkoxyalkyl mit 1 bis 4 Kohlenstoffatomen im Alkylteil und 1 bis 4 Kohlenstoffatomen im Alkoxyteil und/oder Arylalkyloxyalkyl mit 6 bis 10 Kohlenstoffatomen im Arylteil und 1 bis 4 Kohlenstoffatomen im geradkettigen oder verzweigten Alkyloxyteil sowie 1 bis 4 Kohlenstoffatomen im geradkettigen oder verzweigten Alkylteil, wobei der Arylrest einfach oder mehrfach, gleichartig oder verschieden substituiert sein kann durch Halogen und/oder Alkyl mit 1 bis 4 Kohlenstoffatomen,

sowie deren Säureadditionssalze und Metallsalzkomplexe gefunden.

Die Verbindungen der Formel (I) enthalten mindestens ein asymmetrisch substituiertes Kohlenstoffatom. Sie können daher in Form von optisch aktiven Verbindungen vorliegen. Außerdem können diejenigen Verbindungen, in denen A für eine $-CH=CR^1$-Gruppe steht, in Form von geometrischen Isomeren auftreten. Die vorliegende Erfindung betrifft sowohl die reinen Isomeren als auch Isomeren-Gemische.

Weiterhin wurde gefunden, daß sich die neuen substituierten Triazole der Formel (I) sowie deren Säure-Additionssalze und Metallsalz-Komplexe nach mehreren Verfahren herstellen lassen.

So erhält man

(a) substituierte Triazole der Formel

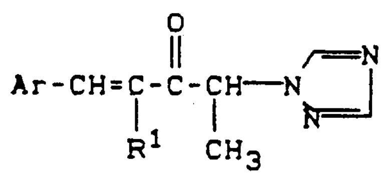

(Ia)

in welcher

Ar und $R^1$ die oben angegebene Bedeutung haben,

wenn man aromatische Aldehyde der Formel

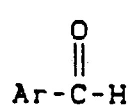

(II)

in welcher

Ar die oben angegebene Bedeutung hat,

mit Triazolylketonen der Formel

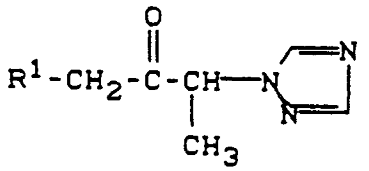

(III)

in welcher

$R^1$ die oben angegebene Bedeutung hat,

gegebenenfalls in Gegenwart eines Verdünnungsmittels und gegebenenfalls in Gegenwart eines Reaktionshilfsmittels umsetzt;

(b) substituierte Triazole der Formel

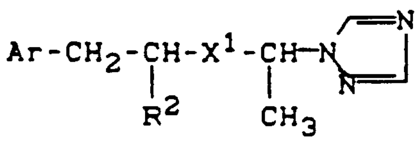

(Ib)

in welcher

$X^1$ für eine der Gruppen

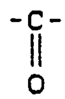 oder 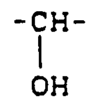

steht und

Ar und $R^2$ die oben angegebene Bedeutung haben,

wenn man substituierte Triazole der Formel

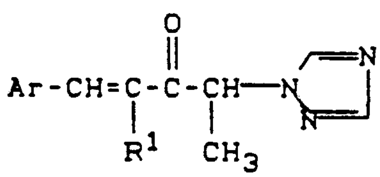

(Ia)

in welcher

Ar und $R^1$ die oben angegebene Bedeutung haben,

mit Wasserstoff in Gegenwart eines Hydrierkatalysators und in Gegenwart eines Verdünnungsmittels hydriert;

(c) substituierte Triazole der Formel

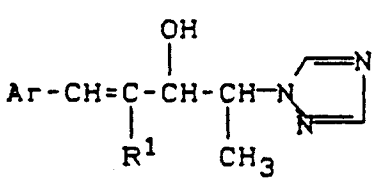

(Ic)

in welcher

Ar und $R^1$ die oben angegebene Bedeutung haben,

wenn man substituierte Triazole der Formel

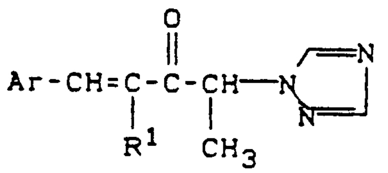

(Ia)

in welcher

Ar und $R^1$ die oben angegebene Bedeutung haben,

mit komplexen Hydriden in Gegenwart eines Verdünnungsmittels reduziert;

(d) substituierte Triazole der Formel

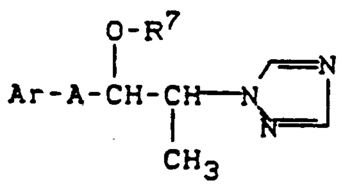

(Id)

in welcher

$R^7$ für jeweils geradkettiges oder verzweigtes Alkyl mit 1 bis 8 Kohlenstoffatomen, Alkenyl mit 3 bis 8 Kohlenstoffatomen oder Alkanoyl mit 1 bis 6 Kohlenstoffatomen im Alkanteil steht, außerdem für Cycloalkyl mit 3 bis 7 Kohlenstoffatomen steht, das einfach oder mehrfach, gleichartig oder verschieden substituiert sein kann durch Halogen, geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen, oder für Aralkyl mit 6 bis 10 Kohlenstoffatomen im Arylteil und 1 bis 4 Kohlenstoffatomen im geradkettigen oder verzweigten Alkylteil steht, wobei jeder der Reste einfach oder mehrfach, gleichartig oder verschieden im Arylteil substituiert sein kann durch Halogen, Cyano, Nitro, jeweils geradkettiges oder verzweigtes Alkyl, Alkoxy oder Alkylthio mit jeweils 1 bis 4 Kohlenstoffatomen und/oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, oder für Aroyl mit 6 bis 10 Kohlenstoffatomen im Arylteil steht, wobei jeder dieser Arylreste einfach oder mehrfach, gleichartig oder verschieden substituiert sein kann durch Halogen, Cyano, Nitro, jeweils geradkettiges oder verzweigtes Alkyl, Alkoxy oder Alkylthio mit jeweils 1 bis 4 Kohlenstoffatomen und/oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen,

Ar und A die oben angegebene Bedeutung haben,

wenn man substituierte Triazole der Formel

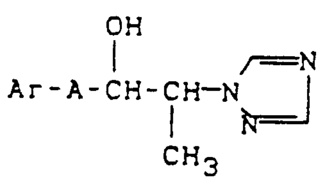

(Ie)

in welcher

Ar und A die oben angegebene Bedeutung haben,

mit Verbindungen der Formel

$R^7$-E (IV )

in welcher

$R^7$ die oben angegebene Bedeutung hat und

E für Chlor, Brom, Iod, Methylsulfonyloxy, p-Methyl-phenyl-sulfonyloxy oder einen Essigsäure- oder Propionsäure-anhydrid-Rest steht,

gegebenenfalls in Gegenwart eines Verdünnungsmittels und gegebenenfalls in Gegenwart eines Reaktionshilfsmittels umsetzt;

(e) substituierte Triazole der Formel

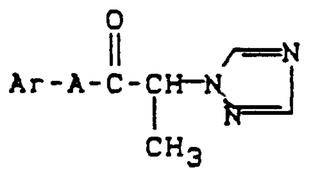

(If)

in welcher

Ar und A die oben angegebene Bedeutung haben,

wenn man substituierte Triazole der Formel

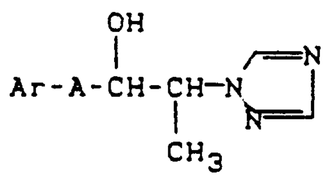

(Ie)

in welcher

Ar und A die oben angegebene Bedeutung haben,

mit einem Oxidationsmittel gegebenenfalls in Gegenwart eines Verdünnungsmittels umsetzt;

(f) substituierte Triazole der Formel

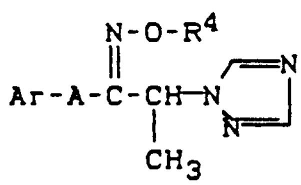

(Ig)

in welcher

Ar, A und $R^4$ die oben angegebene Bedeutung haben,

wenn man substituierte Triazole der Formel

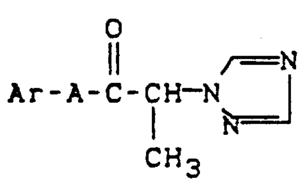

(If)

in welcher

Ar und A die oben angegebene Bedeutung haben,

mit Hydroxylamin-Derivaten der Formel

$H_2N-O-R^4$ (V)

in welcher

$R^4$ die oben angegebene Bedeutung hat,

oder mit deren Säureadditionssalzen gegebenenfalls in Gegenwart eines Verdünnungsmittels sowie gegebenenfalls in Gegenwart eines Reaktionshilfsmittels umsetzt;

(g) substituierte Triazole der Formel

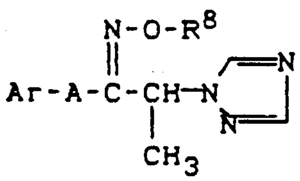

(Ih)

in welcher

Ar und A die oben angegebene Bedeutung haben und

$R^8$ für geradkettiges oder verzweigtes Alkyl mit 1 bis 6 Kohlenstoffatomen oder für Cycloalkyl mit 3 bis 7 Kohlenstoffatomen steht, wobei jeder der Cycloalkylreste einfach oder mehrfach, gleichartig oder verschieden substituiert sein kann durch Halogen, geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen, oder für Aralkyl mit 6 bis 10 Kohlenstoffatomen im Arylteil und 1 bis 4 Kohlenstoffatomen im geradkettigen oder verzweigten Alkylteil steht, wobei jeder der Arylreste einfach oder mehrfach, gleichartig oder verschieden substituiert sein kann durch Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy und/oder Halogenalkylthio mit jeweisl 1 bis 4 Kohlenstoffatomen und 1 bis 9 gleichen oder verschiedenen Halogenatomen,

wenn man substituierte Triazole der Formel

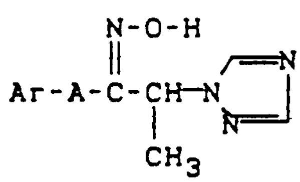

(Ii)

in welcher

Ar und A die oben angegebene Bedeutung haben,

mit Verbindungen der Formel

$R^8-E^1$ (VI)

in welcher

$R^8$ die oben angegebene Bedeutung hat und

$E^1$ für Chlor, Brom, Iod, Methylsulfonyloxy oder p-Methyl-phenyl-sulfonyloxy steht, gegebenenfalls in Gegenwart eines Verdünnungsmittels und gegebenenfalls in Gegenwart eines Reaktionshilfsmittels umsetzt;

(h) substituierte Triazole der Formel

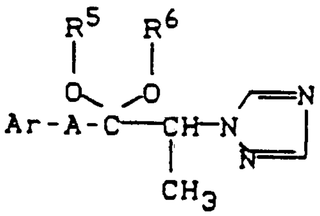

(Ij)

in welcher

Ar, A, $R^5$ und $R^6$ die oben angegebene Bedeutung haben,

wenn man substituierte Triazole der Formel

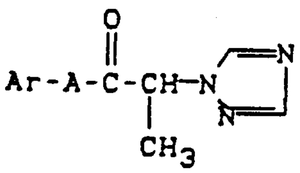

(If)

in welcher

Ar und A die oben angegebene Bedeutung haben,

entweder

$\alpha$) mit Alkoholen der Formeln

$R^5$ - OH (VII) oder $R^6$ - OH (VIII)

in welchen

$R^5$ und $R^6$ die oben angegebenen Bedeutungen haben,

oder

$\beta$) mit Diolen der Formel

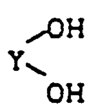

(IX)

in welcher

Y für einen 1,2-Ethandiyl-Rest steht, der einfach bis vierfach, gleichartig oder verschieden substituiert sein kann durch Methyl, Ethyl, n- oder i-Propyl, n-Butyl, Chlormethyl, Trifluormethyl, Methoxymethyl, Ethoxymethyl, Methoxyethyl, Ethoxyethyl und/oder gegebenenfalls im Phenylteil ein- bis dreifach, gleich oder verschieden durch Fluor, Chlor, Brom, Methyl und/oder Ethyl substituiertes Benzyloxymethyl,

gegebenenfalls in Gegenwart eines Verdünnungsmittels und gegebenenfalls in Gegenwart eines Reaktionshilfsmittels umsetzt,

und gegebenenfalls anschließend an die so erhaltenen substituierten Triazole der Formel (I) eine Säure oder ein Metallsalz addiert.

Schließlich wurde gefunden, daß die neuen substituierten Triazole der Formel (I) sowie deren Säureadditionssalze und Metallsalzkomplexe gute fungizide Wirksamkeit besitzen.

Überraschenderweise zeigen die erfindungsgemäßen Stoffe eine erheblich bessere fungizide Wirksamkeit als 1-(4-Chlorphenyl)-2-(1,2,4-triazol-1-yl)-propan-1-ol und 1-(4-Chlorphenyl)-2-(1,2,4-triazol-1-yl )-propan-1-on, welche aus dem Stand der Technik bekannte, chemisch und wirkungsmäßig naheliegende Verbindungen sind.

Die erfindungsgemäßen substituierten Triazole sind durch die Formel (I) allgemein definiert. Bevorzugt sind Verbindungen der Formel (I), in welchen

Ar für Phenyl steht, das einfach bis dreifach, gleichartig oder verschieden substituiert sein kann durch Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Difluormethyl, Fluorchlormethyl, Difluorchlormethyl, Trifluormethoxy, Difluormethoxy, Fluorchlormethoxy, Difluorchlormethoxy, Trifluormethylthio, Methoxycarbonyl, Ethoxycarbonyl, Methoximinomethyl, Ethoximinomethyl, Methoximinoethyl, Ethoximinoethyl, gegebenenfalls ein- bis dreifach, gleich oder verschieden durch Fluor, Chlor, Brom, Methyl und/oder Ethyl substituiertes Phenyl, gegebenenfalls einfach bis dreifach, gleichartig oder verschieden durch Fluor, Chlor, Brom, Methyl und/oder Ethyl substituiertes Phenoxy und/oder durch gegebenenfalls einfach bis dreifach, gleichartig oder verschieden durch Fluor, Chlor, Brom, Methyl und/oder Ethyl substituiertes Benzyloxy,

A für die Gruppen

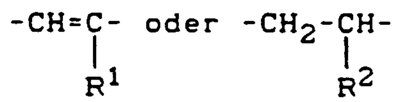

steht und

X für die Gruppen

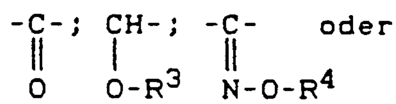

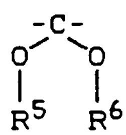

steht, wobei

$R^1$ für Wasserstoff, Methyl, Ethyl, n- oder i-Propyl sowie n-, i-, s- oder t-Butyl steht,

$R^2$ für Wasserstoff, Methyl, Ethyl, n- oder i-Propyl sowie n-, i-, s- oder t-Butyl steht,

$R^3$ für Wasserstoff, Methyl, Ethyl, n- oder i-Propyl, n-, i-, s- oder t-Butyl, für Allyl, für geradkettiges oder verzweigtes Butenyl, für Formyl, Acetyl oder Propionyl steht, ferner für jeweils gegebenenfalls ein- bis dreifach gleich oder verschieden durch Fluor, Chlor, Brom, Methyl, Ethyl und/oder Trifluormethyl substiuiertes Cyclopropyl, Cyclopentyl oder Cyclohexyl steht, und außerdem für Benzyl, Phenethyl oder Benzoyl steht, wobei jeder der drei zuvor genannten Reste im Phenylteil einfach bis dreifach, gleichartig oder verschieden substituiert sein kann durch Fluor, Chlor, Brom, Cyano, Nitro, Methyl, Ethyl, n- oder i-Propyl, n-, i-, s- oder t-Butyl,Methoxy, Ethoxy, n- oder i-Propoxy, Methylthio, Trifluormethyl, Trifluormethoxy und/oder Trifluormethylthio,

$R^4$ für Wasserstoff, Methyl, Ethyl, n- oder i-Propyl, n-, i-, s- oder t-Butyl steht, ferner für jeweils gegebenenfalls ein- bis dreifach, gleich oder verschieden durch Fluor, Chlor, Brom, Methyl, Ethyl und/oder Trifluormethyl substituiertes Cyclopropyl, Cyclopentyl oder Cyclohexyl steht, und außerdem für Benzyl, Phenethyl oder Phenyl steht, wobei jeder der drei zuvor genannten Reste im Phenylteil einfach bis dreifach, gleichartig oder verschieden substituiert sein kann durch Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy und/oder Trifluormethylthio und

$R^5$ und $R^6$ jeweils für Methyl, Ethyl oder Benzyl stehen, wobei der Benzylrest einfach bis dreifach,

gleichartig oder verschieden im Phenylteil substituiert sein kann durch Fluor, Chlor, Brom, Cyano, Nitro, Methyl, Ethyl,n- oder i-Propyl, n-, i-, s- oder t-Butyl, Methoxy, Ethoxy, n- oder i-Propoxy, Methylthio, Trifluormethyl, Trifluormethoxy oder Trifluormethylthio oder

$R^5$ und $R^6$ gemeinsam für einen 1,2-Ethandiylrest stehen, der einfach bis vierfach, gleichartig oder verschieden substituiert sein kann durch Methyl, Ethyl, n- oder i-Propyl, n-Butyl, Chlormethyl, Trifluormethyl, Methoxyethyl, Ethoxymethyl, Methoxyethyl, Ethoxyethyl und/oder gegebenenfalls im Phenylteil ein- bis dreifach, gleich oder verschieden durch Fluor, Chlor, Brom, Methyl und/oder Ethyl substituiertes Benzyloxymethyl.

Bevorzugte erfindungsgemäße Verbindungen sind auch Additionsprodukte aus Säuren und denjenigen substituierten Triazolen der Formel (I), in denen die Substituenten Ar, A und X die Bedeutungen haben, die bereits vorzugsweise für diese Substituenten genannt wurden.

Zu den Säuren die addiert werden können, gehören vorzugsweise Halogenwasserstoffsäuren, wie z.B Chlorwasserstoffsäure und Bromwasserstoffsäure, insbesondere Chlorwasserstoffsäure, ferner Phosphorsäure, Salpetersäure, Schwefelsäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, Sulfonsäuren, wie z.B. p-Toluolsulfonsäure und 1,5-Naphthalindisulfonsäure sowie Saccharin oder Thiosaccharin.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und denjenigen substituierten Triazolen der Formel (I), in denen die Substituenten Ar, A und X die Bedeutungen haben, die bereits vorzugsweise für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu pflanzenverträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, Salpetersäure und Schwefelsäure.

Besonders bevorzugt sind Verbindungen der Formel (I), in welchen

Ar für Phenyl steht, das einfach oder zweifach, gleichartig oder verschieden substituiert sein kann durch Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Trifluormethyl, Difluormethyl, Fluorchlormethyl, Difluorchlormethyl, Trifluormethoxy, Difluormethoxy, Fluorchlormethoxy, Difluorchlormethoxy, Trifluormethylthio, Methoximinomethyl, Ethoximinomethyl, Methoximinoethyl oder Ethoximinoethyl, gegebenenfalls durch Chlor einfach oder zweifach substituiertes Phenyl, gegebenenfalls einfach oder zweifach durch Chlor substituiertes Phenoxy und/oder gegebenenfalls einfach oder zweifach durch Chlor substituiertes Benzyloxy,

A für die Gruppen

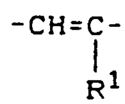 oder 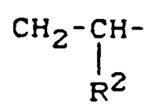

steht und

X für die Gruppen

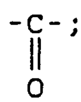 ; 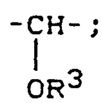 ; 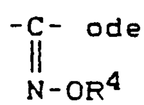 oder 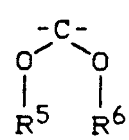

steht,

wobei

$R^1$ für Wasserstoff oder Methyl steht,

$R^2$ für Wasserstoff oder Methyl steht,

$R^3$ für Wasserstoff, Methyl, Ethyl, Allyl, Acetyl, Propionyl, für Cyclohexyl, gegebenenfalls einfach oder zweifach, gleichartig oder verschieden durch Fluor, Chlor und/oder Trifluormethoxy substituiertes Benzyl oder Benzoyl steht,

$R^4$ für Wasserstoff, Methyl, Ethyl, Cyclohexyl, gegebenenfalls ein- oder zweifach durch Chlor oder Nitro substituiertes Benzyl, Phenethyl oder Phenyl steht und

$R^5$ und $R^6$ jeweils für Methyl, Ethyl oder Benzyl stehen oder gemeinsam für einen gegebenenfalls durch Methyl oder Chlormethyl substituierten 1,2-Ethandiylrest stehen.

Im einzelnen seien außer den bei den Herstellungsbeispielen genannten Verbindungen die folgenden substituierten Triazole der allgemeinen Formel (I) genannt:

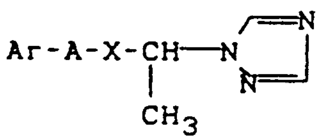 (I)

| Ar | A | X |
|---|---|---|
| 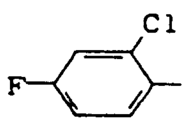 | 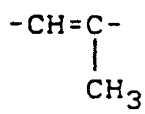 | 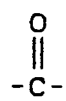 |
| 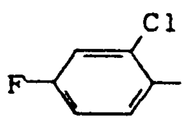 | 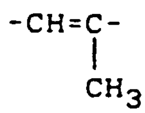 | 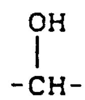 |
| 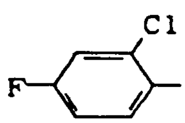 | 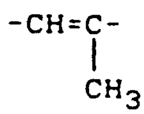 | 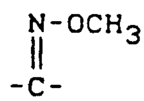 |

| Ar | A | X |
|---|---|---|
| 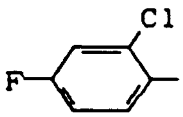 | 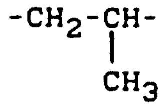 | 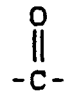 |
| 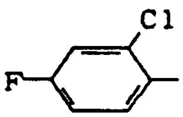 | 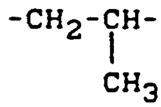 | 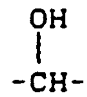 |
| 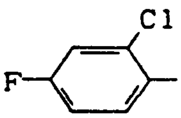 | 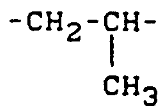 | 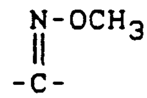 |
| 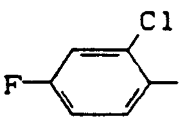 | 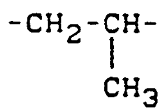 | 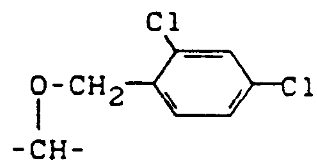 |
| 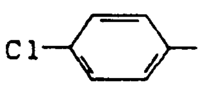 | 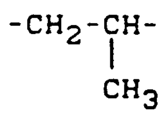 | 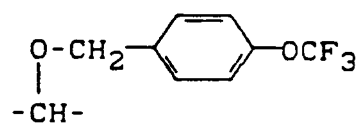 |
| 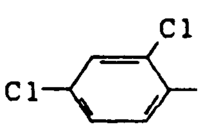 | 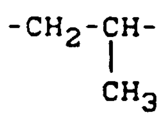 | 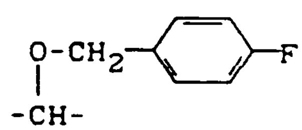 |

| Ar | A | X |
|---|---|---|
| 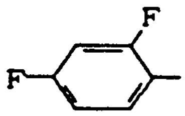 | 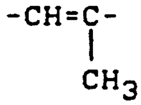 | 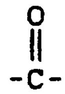 |
| 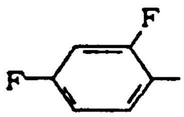 | 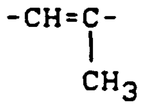 | 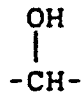 |
| 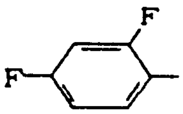 | 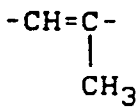 | 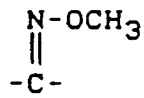 |
| 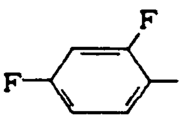 | 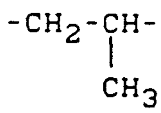 | 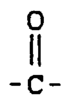 |
| 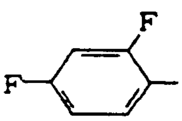 | 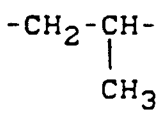 | 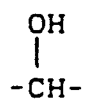 |
| 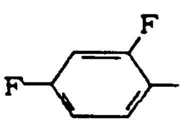 | 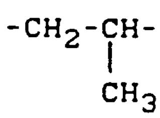 | 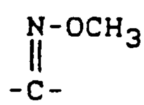 |

| Ar | A | X |
|---|---|---|
| 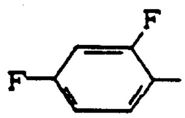 | 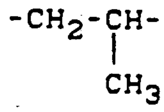 | 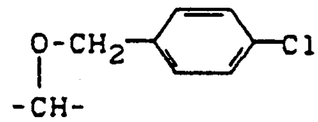 |
| 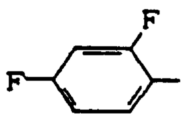 | 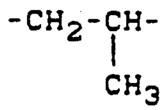 | 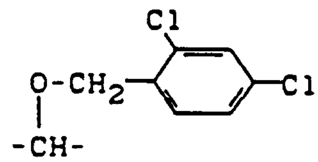 |
| 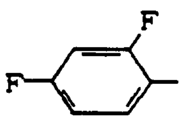 | 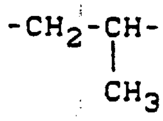 | 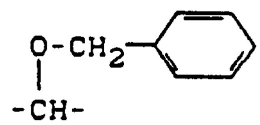 |
| 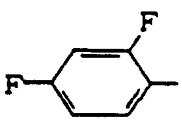 | 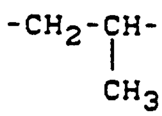 | 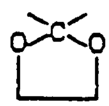 |
| 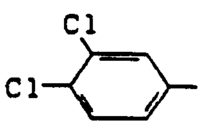 | 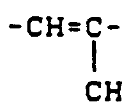 | 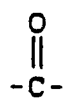 |
| 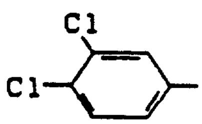 | 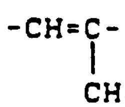 | 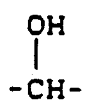 |

| Ar | A | X |
|---|---|---|
| 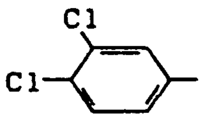 | 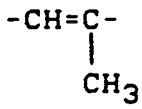 | 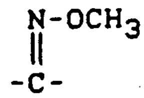 |
| 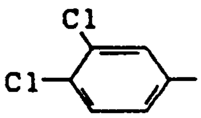 | 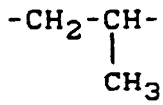 | 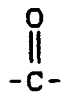 |
| 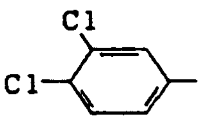 | 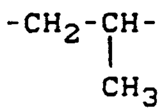 | 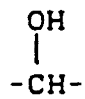 |
| 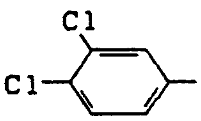 | 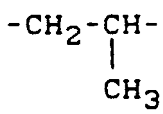 | 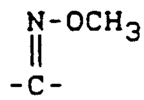 |
| 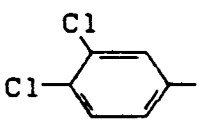 | 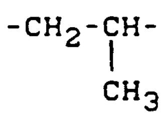 | 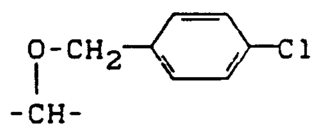 |
| 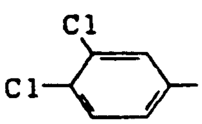 | 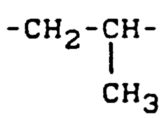 | 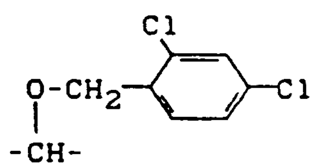 |

| Ar | A | X |
|---|---|---|
| 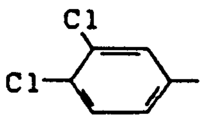 | 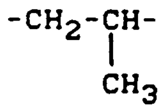 | 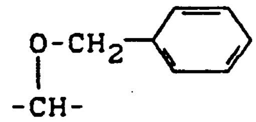 |
| 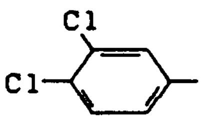 | 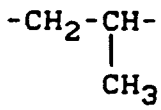 | 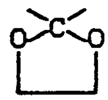 |
| 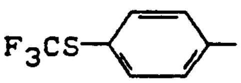 | 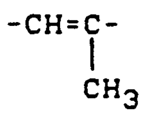 | 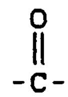 |
| 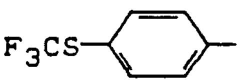 | 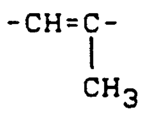 | 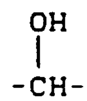 |
| 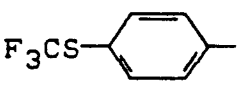 | 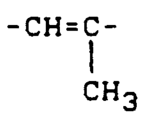 | 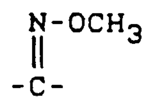 |
| 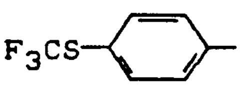 | 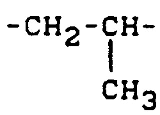 | 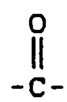 |

| Ar | A | X |
|---|---|---|
| 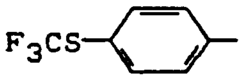 | 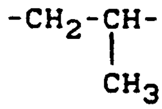 | 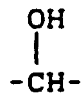 |
| 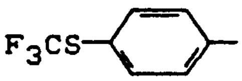 | 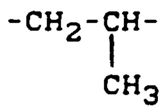 | 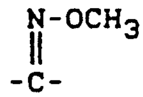 |
| 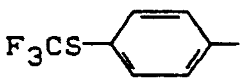 | 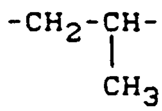 | 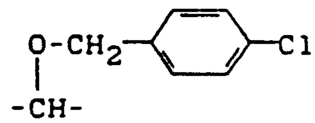 |
| 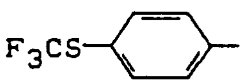 | 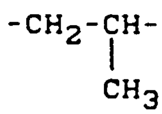 | 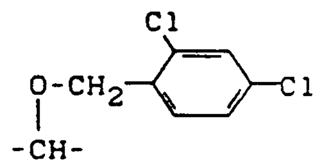 |
| 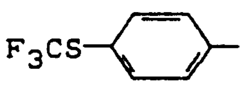 | 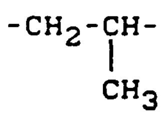 | 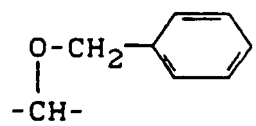 |
| 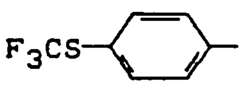 | 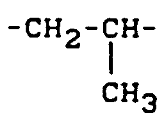 | 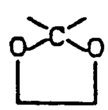 |

| Ar | A | X |
|---|---|---|
| 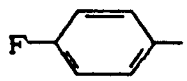 | 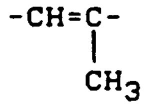 | 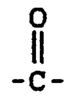 |
| 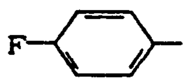 | 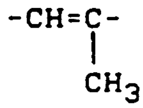 | 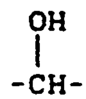 |
| 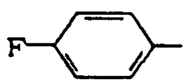 | 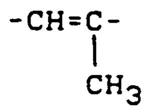 | 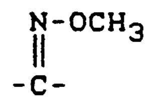 |
| 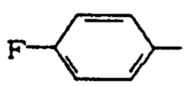 | 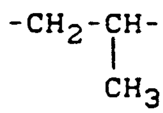 | 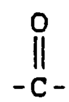 |
| 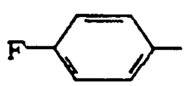 | 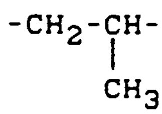 | 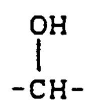 |
| 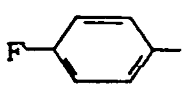 | 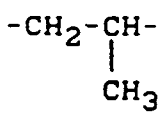 | 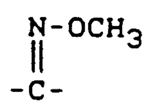 |

| Ar | A | X |
|---|---|---|
| 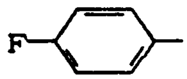 | 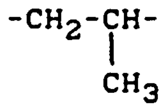 | 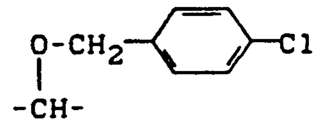 |
| 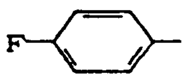 | 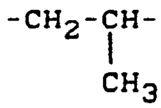 | 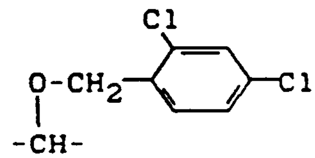 |
| 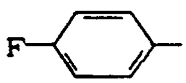 | 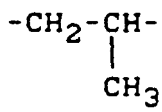 | 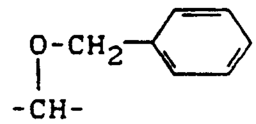 |
| 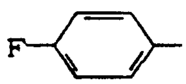 | 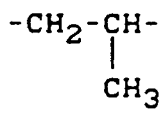 | 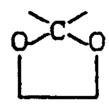 |
| 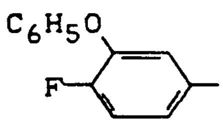 | 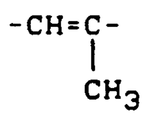 | 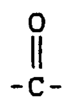 |
| 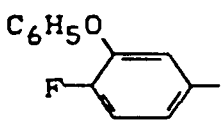 | 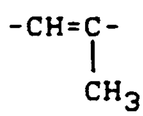 | 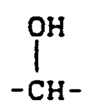 |

| Ar | A | X |
|---|---|---|
| 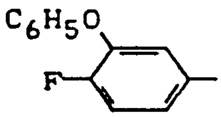 | 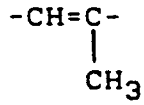 | 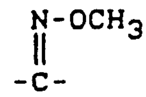 |
| 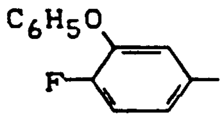 | 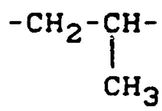 | 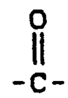 |
| 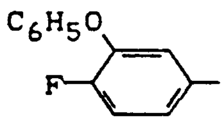 | 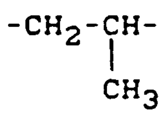 | 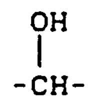 |
| 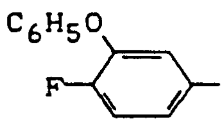 | 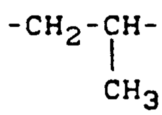 | 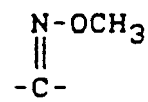 |
| 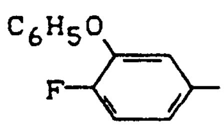 | 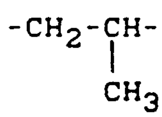 | 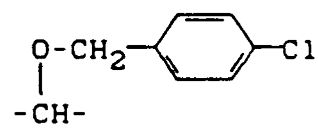 |
| 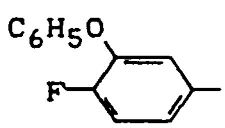 | 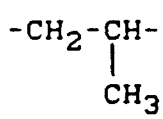 | 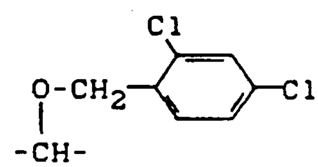 |

| Ar | A | X |
|---|---|---|
| 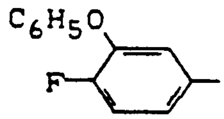 | 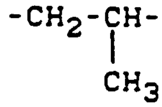 | 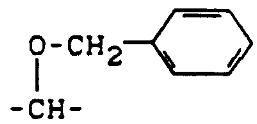 |
| 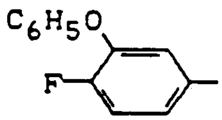 | 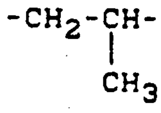 | 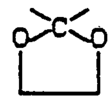 |
| 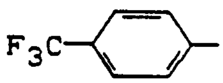 | 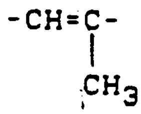 | 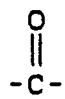 |
| 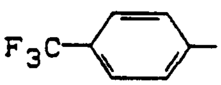 | 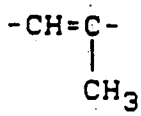 | 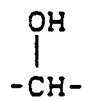 |
| 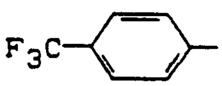 | 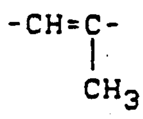 | 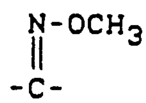 |
| 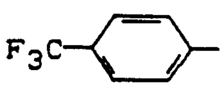 | 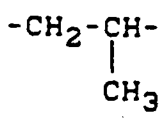 | 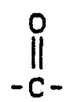 |

| Ar | A | X |
|---|---|---|
| 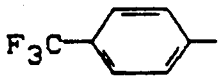 | 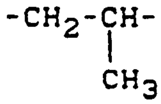 | 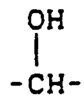 |
| 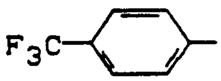 | 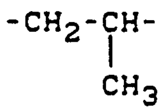 | 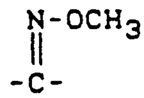 |
| 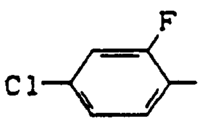 | 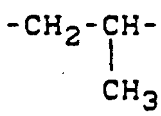 | 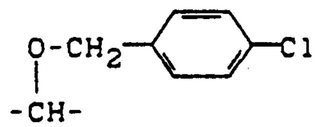 |
| 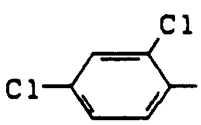 | 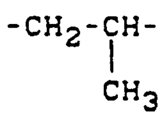 | 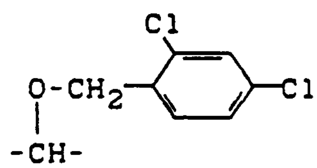 |
| 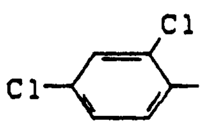 | 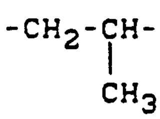 | 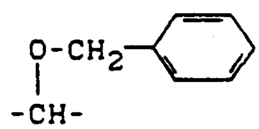 |
| 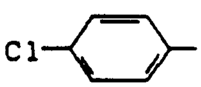 | 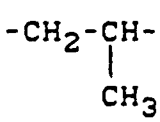 | 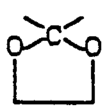 |

| Ar | A | X |
|---|---|---|
| 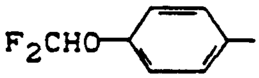 | 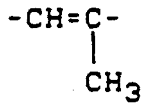 | 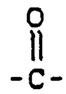 |
| 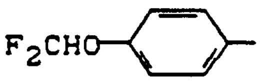 | $-CH=CH-$ | 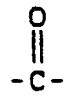 |
| 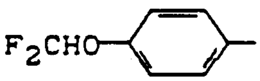 | $-CH_2-CH_2-$ | 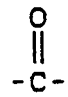 |
| 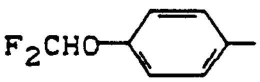 | $-CH_2-CH_2$ | 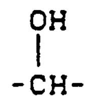 |
| 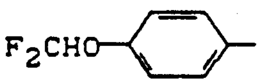 | $-CH_2-CH_2-$ | 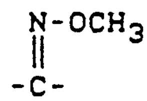 |
| 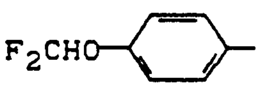 | $-CH_2-CH_2-$ | 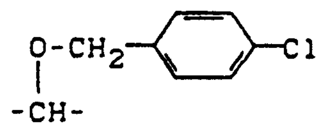 |
| 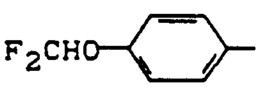 | 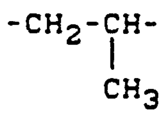 | 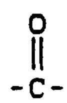 |

| Ar | A | X |
|---|---|---|
| 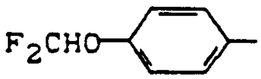 | 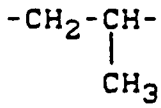 | 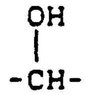 |
| 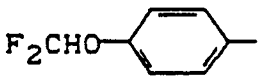 | 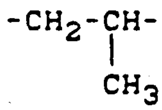 | 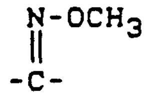 |
| 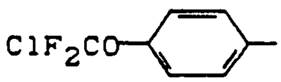 | 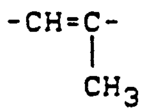 | 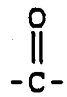 |
| 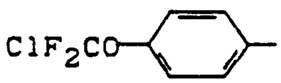 | $-CH=CH-$ | 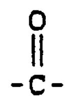 |
| 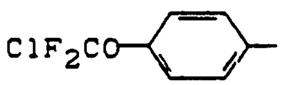 | $-CH_2-CH_2-$ | 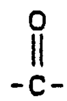 |
| 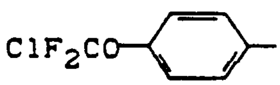 | $-CH_2-CH_2-$ | 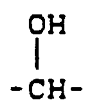 |
| 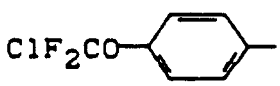 | $-CH_2-CH_2-$ | 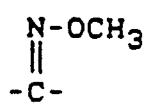 |

| Ar | A | X |
|---|---|---|
| 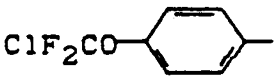 | $-CH_2-CH_2-$ | 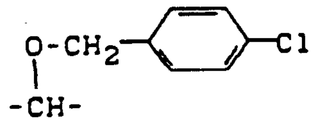 |
| 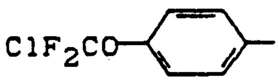 | 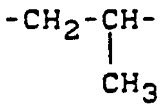 | 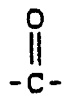 |
| 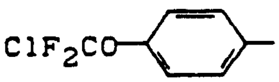 | 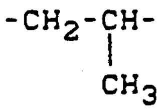 | 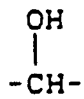 |
| 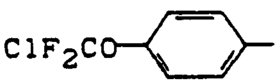 | 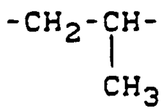 | 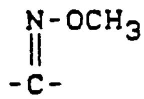 |
| 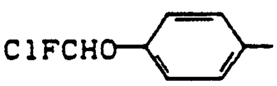 | 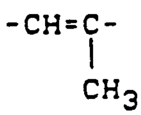 | 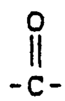 |
| 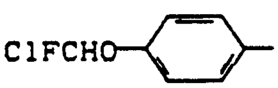 | $-CH=CH-$ | 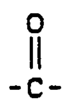 |

| Ar | A | X |
|---|---|---|
| 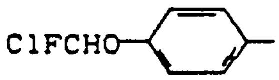 | $-CH_2-CH_2-$ | 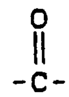 |
| 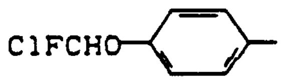 | $-CH_2-CH_2-$ | 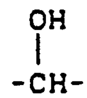 |
| 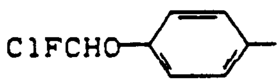 | $-CH_2-CH_2-$ | 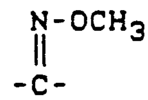 |
| 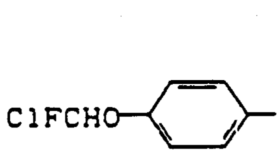 | $-CH_2-CH_2-$ | 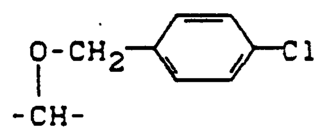 |
| 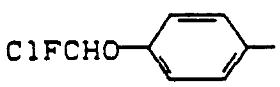 | 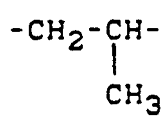 | 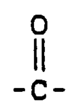 |
| 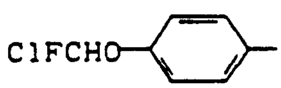 | 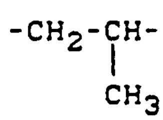 | 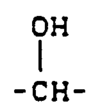 |
| 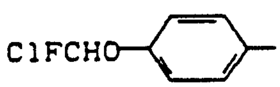 | 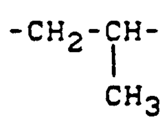 | 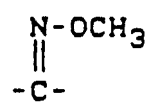 |

Verwendet man beispielsweise 4-Chlorbenzaldehyd und 3-(1,2,4-Triazol-1-yl)-butan-2-on als Ausgangsstoffe, so läßt sich der Ablauf des erfindungsgemäßen Verfahrens (a) durch das folgende Formelschema darstellen:

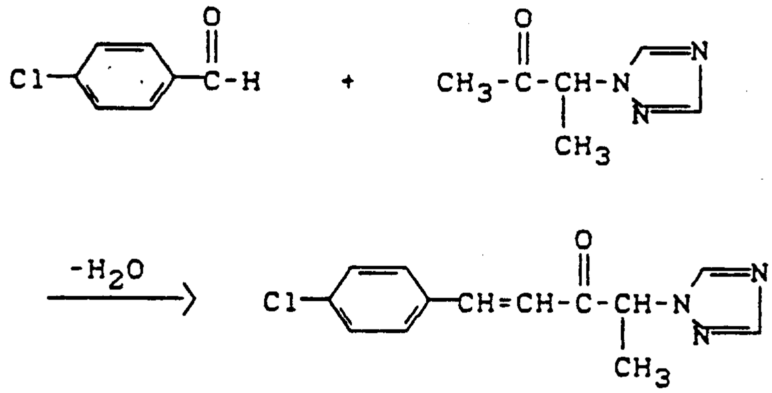


Verwendet man beispielsweise 1-(4-Chlorphenyl)-4-(1,2,4-triazol-1-yl)-pent-1-en-3-on und Wasserstoff als Ausgangsverbindungen und Raney-Nickel als Hydrierkatalysator, so läßt sich der Ablauf des erfindungsgemäßen Verfahrens (b) durch das folgende Formelschema darstellen:

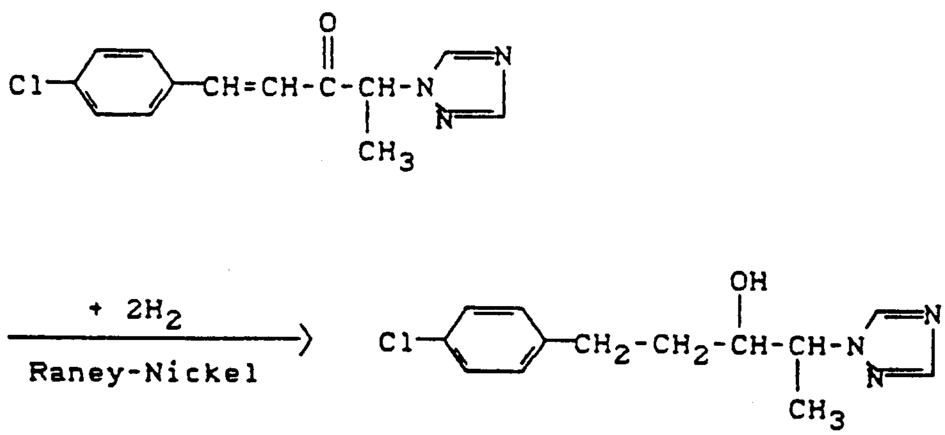


Verwendet man beispielsweise 1-(4-Chlorphenyl)-4-(1,2,4-triazol-1-yl)-pent-1-en-3-on als Ausgangsverbindung und Natriumborhydrid als komplexes Hydrid, so läßt sich der Ablauf des erfindungsgemäßen Verfahrens (c) durch das folgende Formelschema darstellen:

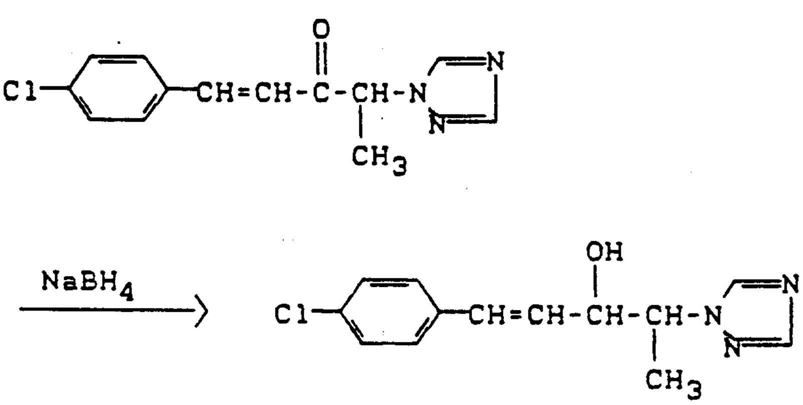


Verwendet man beispielsweise 1-(4-Chlorphenyl)-4-(1,2,4-triazol-1-yl)-pent-1-en-3-ol und 4-Chlorbenzylchlorid als Ausgangsstoffe, so läßt sich der Ablauf des erfindungsgemäßen Verfahrens (d) durch das folgende Formelschema darstellen:

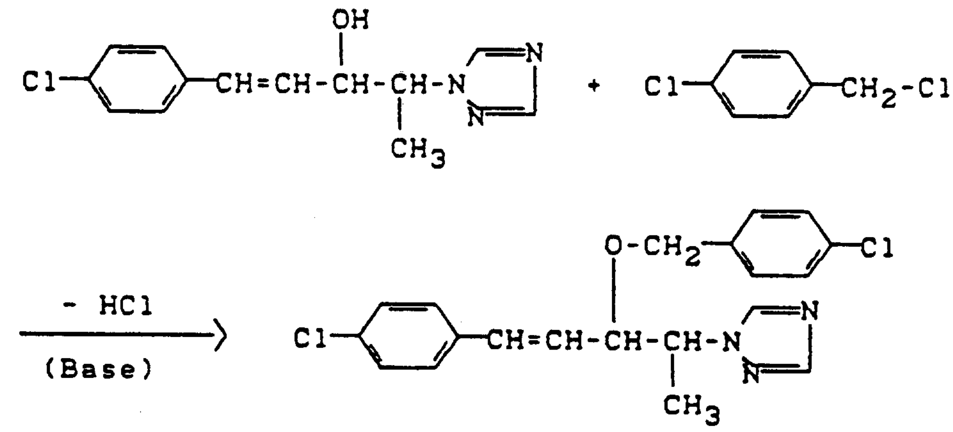


Verwendet man beispielsweise 1-(4-Chlorphenyl)-4-(1,2,4-triazol-1-yl)-pentan-3-ol als Ausgangsverbindung und Dimethylsulfoxid als Oxidationsmittel, so läßt sich der Ablauf des erfindungsgemäßen Verfahrens (e) durch das folgende Formelschema darstellen:

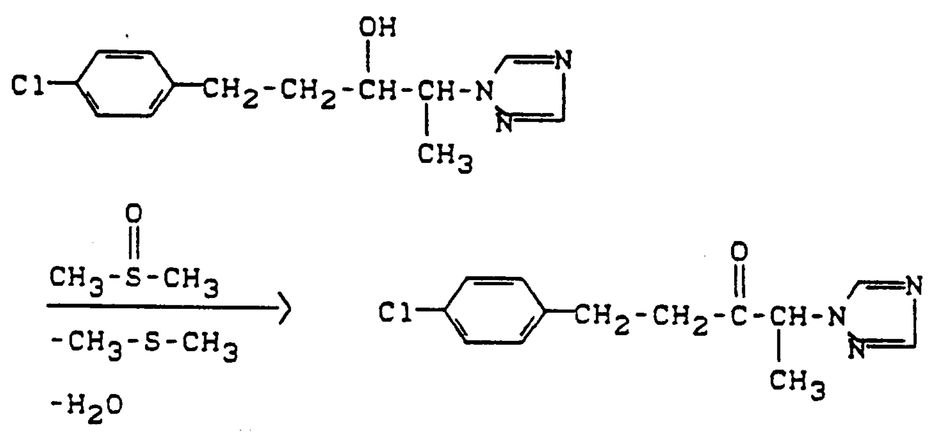


Verwendet man beispielsweise 1-(4-Chlorphenyl)-4-(1,2,4-triazol-1-yl)-pent-1-en-3-on und O-(2-Chlorbenzyl)-hydroxylamin als Ausgangsstoffe, so läßt sich der Ablauf des erfindungsgemäßen Verfahrens (f) durch das folgende Formelschema darstellen:

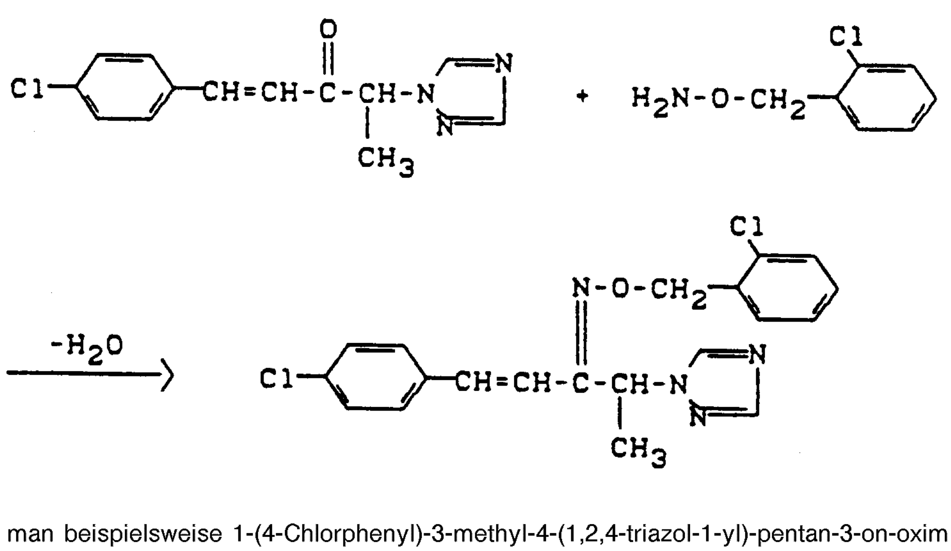


Verwendet man beispielsweise 1-(4-Chlorphenyl)-3-methyl-4-(1,2,4-triazol-1-yl)-pentan-3-on-oxim und Dimethylsulfat als Ausgangsstoffe, so läßt sich der Ablauf des erfindungsgemäßen Verfahrens (g) durch das

folgende Formelschema darstellen:

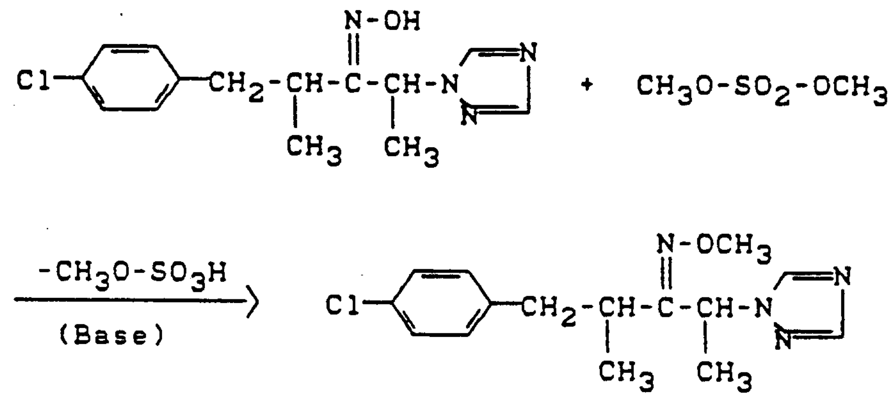


Verwendet man beispielsweise 1-(2,4-Dichlorphenyl)-4-(1,2,4-triazol-1-yl)-pentan-3-on und 3-Chlorpropan-1,2-diol als Ausgangsstoffe, so läßt sich der Ablauf des erfindungsgemäßen Verfahrens (h) durch das folgende Formelschema darstellen:

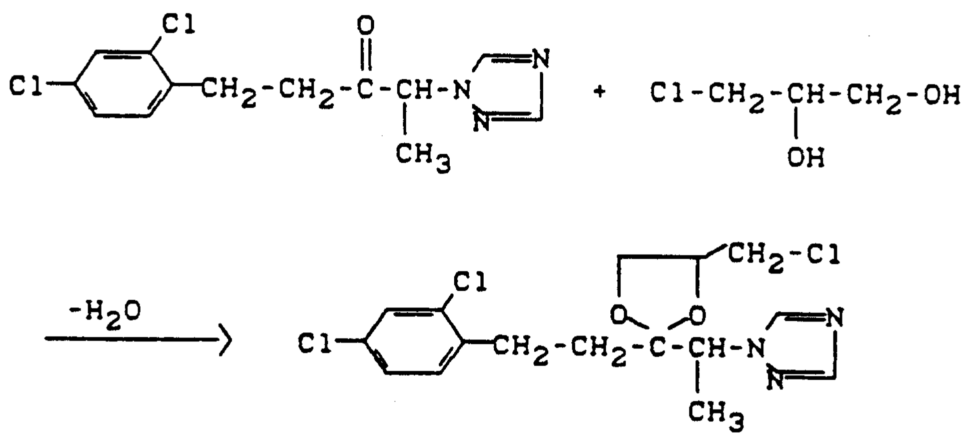


Die zur Durchführung des erfindungsgemäßen Verfahrens (a) als Ausgangsstoffe benötigten aromatischen Aldehyde sind durch die Formel (II) allgemein definiert. In dieser Formel (II) steht Ar vorzugsweise für diejenigen Reste, die bereits im Zusammenhang mit der Beschreibung der erfindungsgemäßen Stoffe der Formel (I) als bevorzugt für diesen Substituenten genannt wurden.

Die aromatischen Aldehyde der Formel (II) sind allgemein bekannte Verbindungen der organischen Chemie (vgl. z.B. DE-A 30 17 339 und J. Med. Chem. 16, 1399 [1973]).

Die zur Durchführung des erfindungsgemäßen Verfahrens (a) weiterhin als Ausgangsstoffe benötigten Triazolylketone sind durch die Formel (III) allgemein definiert. In dieser Formel (III) steht $R^1$ vorzugsweise für diejenigen Reste, die bereits im Zusammenhang mit der Beschreibung der erfindungsgemäßen Stoffe der Formel (I) als bevorzugt für diesen Substituenten genannt wurden.

Die Triazolylketone der Formel (III) sind bekannt (vgl. DE-A 24 31 407).

Die zur Durchführung der erfindungsgemäßen Verfahren (b) und (c) als Ausgangsstoffe benötigten substituierten Triazole sind durch die Formel (Ia) allgemein definiert. In dieser Formel (Ia) stehen $R^1$ und Ar vorzugsweise für diejenigen Reste, die bereits im Zusammenhang mit der Beschreibung der erfindungsgemäßen Stoffe der Formel (I) als bevorzugt für diese Substituenten genannt wurden.

Die substituierten Triazole der Formel (Ia) sind erfindungsgemäße Verbindungen und lassen sich nach dem erfindungsgemäßen Verfahren (a) herstellen.

Die zur Durchführung der erfindungsgemäßen Verfahren (d) und (e) als Ausgangsstoffe benötigten substituierten Triazole sind durch die Formel (Ie) allgemein definiert. In dieser Formel (Ie) stehen Ar und A vorzugsweise für diejenigen Reste, die bereits im Zusammenhang mit der Beschreibung der erfindungsgemäßen Stoffe der Formel (I) als bevorzugt für diese Substituenten genannt wurden.

Die substituierten Triazole der Formel (Ie) sind erfindungsgemäße Verbindungen und lassen sich nach den erfindungsgemäßen Verfahren (b) oder (c) herstellen.

Die zur Durchführung des erfindungsgemäßen Verfahrens (d) weiterhin als Ausgangsstoffe benötigten Verbindungen sind durch die Formel (IV) allgemein definiert. In dieser Formel (IV) steht $R^7$ vorzugsweise für diejenigen Reste, die bereits im Zusammenhang mit der Beschreibung der erfindungsgemäßen Stoffe der Formel (I) als bevorzugt für den Substituenten $R^3$ genannt wurden, mit Ausnahme des Wasserstoffrestes.

Die Verbindungen der Formel (IV) sind allgemein bekannte Verbindungen der organischen Chemie.

Die zur Durchführung der erfindungsgemäßen Verfahren (f) und (h) als Ausgangsstoffe benötigten substituierten Triazole sind durch die Formel (If) allgemein definiert. In dieser Formel (If) stehen Ar und A vorzugsweise für diejenigen Reste, die bereits im Zusammenhang mit der Beschreibung der erfindungsgemäßen Stoffe der Formel (I) als bevorzugt für diese Substituenten genannt wurden.

Die substituierten Triazole der Formel (If) sind erfindungsgemäße Verbindungen und lassen sich nach den erfindungsgemäßen Verfahren (a), (b) oder (e) herstellen.

Die zur Durchführung des erfindungsgemäßen Verfahrens (f) weiterhin als Ausgangsstoffe benötigten Hydroxylamin-Derivate sind durch die Formel (V) allgemein definiert. In dieser Formel (V) steht $R^4$ vorzugsweise für diejenigen Reste, die bereits im Zusammenhang mit der Beschreibung der erfindungsgemäßen Stoffe der Formel (I) als bevorzugt für diese Substituenten genannt wurden.

Die Hydroxylamin-Derivate der Formel (V) sowie deren Säureadditionssalze, wie beispielsweise deren Hydrochloride oder Hydroacetate sind allgemein bekannte Verbindungen der organischen Chemie.

Die zur Durchführung des erfindungsgemäßen Verfahrens (g) als Ausgangsstoffe benötigten substituierten Triazole sind durch die Formel (Ii) allgemein definiert. In dieser Formel (Ii) stehen Ar und A vorzugsweise für diejenigen Reste, die bereits im Zusammenhang mit der Beschreibung der erfindungsgemäßen Stoffe der Formel (I) als bevorzugt für diese Substituenten genannt wurden.

Die substituierten Triazole der Formel (Ii) sind erfindungsgemäße Verbindungen und lassen sich nach dem erfindungsgemäßen Verfahren (f) herstellen.

Die zur Durchführung des erfindungsgemäßen Verfahrens (g) weiterhin als Ausgangsstoffe benötigten Verbindungen sind durch die Formel (VI) allgemein definiert. In dieser Formel (VI) steht $R^8$ vorzugsweise für diejenigen Reste, die bereits im Zusammenhang mit der Beschreibung der erfindungsgemäßen Stoffe der Formel (I) als bevorzugt für den Substituenten $R^4$ genannt wurden mit Ausnahme des Wasserstoffrestes und des gegebenenfalls substituierten Arylrestes.

Die Verbindungen der Formel (VI) sind allgemein bekannte Verbindungen der organischen Chemie.

Die zur Durchführung des erfindungsgemäßen Verfahrens (h) weiterhin als Ausgangsstoffe benötigten Alkohole und Diole sind durch die Formeln (VII), (VIII) und (IX) allgemein definiert.

Bevorzugt sind diejenigen Alkohole der Formeln (VII) und (VIII), in denen

$R^5$ beziehungsweise $R^6$ jeweils für Methyl, Ethyl oder Benzyl stehen, wobei der Benzylrest einfach bis dreifach, gleichartig oder verschieden im Phenylteil substituiert sein kann durch Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy oder Trifluormethylthio.

Besonders bevorzugt sind diejenigen Alkohole der Formeln (VII) und (VIII), in denen $R^5$ beziehungsweise $R^6$ für jeweils Methyl, Ethyl oder Benzyl stehen.

Bevorzugt sind schließlich auch diejenigen Diole der Formel (IX), in denen Y für einen gegebenenfalls durch Methyl oder Chlormethyl substituierten 1,2-Ethandiyl-Rest steht.

Die Alkohole der Formeln (VII) und (VIII) sowie die Diole der Formel (IX) sind allgemein bekannte Verbindungen der organischen Chemie.

Als Verdünnungsmittel zur Durchführung des erfindungsgemäßen Verfahrens (a) kommen inerte organische Lösungsmittel in Frage. Vorzugsweise verwendbar sind aliphatische, alicyclische und aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder - diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Sulfoxide, wie Dimethylsulfoxid, Alkohole wie Methanol, Ethanol oder Propanol oder heterocyclische Basen, wie Pyridin sowie gegebenenfalls auch deren Gemische mit Wasser.

Das erfindungsgemäße Verfahren (a) kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}/C_{15}$-alkylammoniumchlorid, Dibenzyl-dimethylammoniummethylsulfat, Dimethyl-$C_{12}/C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid oder Tris-[2-(2-methoxy-ethoxy)-ethyl]-amin.

Das erfindungsgemäße Verfahren (a) wird vorzugsweise in Gegenwart eines geeigneten Reaktionshilfsmittels durchgeführt. Als solche kommen alle üblicherweise verwendbaren anorganischen und organischen Basen oder Säuren in Frage. Vorzugsweise verwendet man Alkalimetallhydroxide, -alkoholate, -carbonate oder -hydrogencarbonate, wie beispielsweise Natriumhydroxid, Natriummethylat, Natriumethylat, Kalium-t-butylat, Natriumcarbonat, Kaliumcarbonat oder Natriumhydrogencarbonat oder auch Amine, wie beispielsweise Triethylamin, N,N-Dimethylanilin, Pyridin, Piperidin, N,N-Dimethylaminopyridin, Diazabicyclooctan (DABCO), Diazabicyclononen (DBN) oder Diazabicycloundecen (DBU) oder Säuren wie beispielsweise Salzsäure oder Essigsäure oder auch Gemische von Säuren und Basen der oben genannten Art.

Die Reaktionstemperaturen können bei der Durchführung des erfindungsgemäßen Verfahrens (a) in einem größeren Bereich variiert werden. Im allgemeinen arbeitet man bei Temperaturen zwischen - 50 °C und + 200 °C, vorzugsweise bei Temperaturen zwischen 0 °C und 150 °C.

Zur Durchführung des erfindungsgemäßen Verfahrens (a) setzt man pro Mol an aromatischem Aldehyd der Formel (II) im allgemeinen 1,0 bis 1,5 Mol, vorzugsweise 1,0 bis 1,2 Mol an Triazolylketon der Formel (III) und gegebenenfalls 0,01 bis 1,0 Mol, vorzugsweise 0,1 bis 0,5 Mol an Reaktionshilfsmittel ein. Die Reaktionsdurchführung, Aufarbeitung und Isolierung der Reaktionsprodukte erfolgt nach allgemein üblichen Methoden (vgl. auch die Herstellungsbeispiele).

Als Hydrierkatalysatoren zur Durchführung des erfindungsgemäßen Verfahrens (b) kommen alle üblichen Edelmetall-, Edelmetalloxid- und Raney-Hydrierkatalysatoren infrage. Mit besonderem Vorzug verwendet man Raney-Nickel als Hydrierkatalysator.

Als Verdünnungsmittel zur Durchführung des erfindungsgemäßen Verfahrens (b) kommen ebenfalls inerte organische Lösungsmittel infrage. Vorzugsweise verwendet man die bei Verfahren (a) genannten Verdünnungsmittel oder Ester wie Essigsäureethylester.

Die Reaktionstemperaturen können bei der Durchführung des erfindungsgemäßen Verfahrens (b) in einem größeren Bereich variiert werden. Im allgemeinen arbeitet man bei Temperaturen zwischen 0 °C und 200 °C, vorzugsweise bei Temperaturen zwischen 20 °C und 150 °C.

Das erfindungsgemäße Verfahren (b) wird üblicherweise unter Druck durchgeführt. Vorzugsweise arbeitet man bei einem Wasserstoffdruck zwischen 1 und 200 bar insbesondere zwischen 10 und 100 bar.

Zur Durchführung des erfindungsgemäßen Verfahrens (b) setzt man pro Mol an substituiertem Triazol der Formel (Ia) im allgemeinen 0,001 bis 0,5 Mol, vorzugsweise 0,01 bis 0,1 Mol an Hydrierkatalysator sowie einen Überschuß an Wasserstoff ein.
Die Reaktionsdurchführung, Aufarbeitung und Isolierung der Reaktionsprodukte erfolgt nach allgemein üblichen Methoden.

Dabei kommt es in Abhängigkeit von der Art der Substituenten in der Ausgangsverbindung der Formel (Ia) sowie in Abhängigkeit von den Reaktionsbedingungen (Temperatur, Katalysatormenge, Wasserstoffdruck, Reaktionsdauer) zunächst zu einer Hydrierung der C=C-Doppelbindung im Molekül und anschließend gegebenenfalls zu einer Weiterhydrierung der C=O-Doppelbindung, so daß entweder 1-Aryl-4-triazolylpentan-3-one oder 1-Aryl-4-triazolylpentan-3-ole als Endprodukte erhalten werden können (vgl. auch die Herstellungsbeispiele).

Das erfindungsgemäße Verfahren (c) wird in Gegenwart eines üblichen komplexen Hydrids als Reduktionsmittel durchgeführt. Mit besonderm Vorzug verwendet man Natriumborhydrid, Natriumcyanoborhydrid oder Lithiumborhydrid, gegebenenfalls in Gegenwart von Calciumchlorid, wobei sich im Reaktionsgemisch auch komplexe Calciumborhydride bilden können.

Als Verdünnungsmittel zur Durchführung des erfindungsgemäßen Verfahrens (c) kommen inerte organische Lösungsmittel infrage. Vorzugsweise verwendet man Ether, wie Diethylether, Dioxan, Tetrahydrofuran oder Ethylenglykoldimethyl- oder -diethylether oder Alkohole, wie Methanol, Ethanol sowie n- oder i-Propanol gegebenenfalls auch in Mischung mit Wasser.

Die Reaktionstemperaturen können bei der Durchführung des erfindungsgemäßen Verfahrens (c) in einem größeren Bereich variiert werden. Im allgemeinen arbeitet man bei Temperaturen zwischen - 100 °C und + 200 °C, vorzugsweise bei Temperaturen zwischen - 50 °C und + 50 °C.

Zur Durchführung des erfindungsgemäßen Verfahrens (c) setzt man pro Mol an substituiertem Triazol der Formel (Ia) im allgemeinen 0,1 bis 1,5 Mol, vorzugsweise 0,25 bis 1,0 Mol an komplexem Hydrid und gegebenenfalls 0,1 bis 1,5 Mol, vorzugsweise 0,25 bis 1,0 Mol an Calciumchlorid ein.
Die Reaktionsdurchführung, Aufarbeitung und Isolierung der Reaktionsprodukte erfolgt nach allgemein üblichen Methoden (vgl. auch die Herstellungsbeispiele).

Als Verdünnungsmittel zur Durchführung des erfindungsgemäßen Verfahrens (d) kommen inerte organische Lösungsmittel infrage. Vorzugsweise verwendbar sind aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder - diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (d) kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}$/$C_{15}$-alkylammoniumchlorid, Dibenzyl-dimethylammoniummethylsulfat, Dimethyl-$C_{12}$/$C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid.

Das erfindungsgemäße Verfahren (d) wird vorzugsweise in Gegenwart eines geeigneten Reaktionshilfsmittels durchgeführt. Als solche kommen alle üblicherweise verwendbaren anorganischen und organischen Basen in Frage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Bei den Reaktionen nach dem erfindungsgemäßen Verfahren (d) kann es auch von Vorteil sein, geringe Mengen üblicher Katalysatoren, wie beispielsweise Kaliumiodid, zuzusetzen.

Die Reaktionstemperaturen können bei der Durchführung des erfindungsgemäßen Verfahrens (d) in einem größeren Bereich variiert werden. Im allgemeinen arbeitet man bei Temperaturen zwischen - 50 °C und 200 °C, vorzugsweise bei Temperaturen zwischen 0 °C und 100 °C.

Zur Durchführung des erfindungsgemäßen Verfahrens (d) setzt man pro Mol an substituiertem Triazol der Formel (Ie) im allgemeinen 1,0 bis 5,0 Mol, vorzugsweise 1,0 bis 1,5 Mol an einer Verbindung der Formel (IV) und gegebenenfalls 1,0 bis 3,0 Mol, vorzugsweise 1,0 bis 1,2 Mol an Reaktionshilfsmittel ein.

Die Reaktionsdurchführung, Aufarbeitung und Isolierung der Reaktionsprodukte erfolgt nach allgemein üblichen Methoden (vgl. auch die Herstellungsbeispiele).

Als Oxidationsmittel zur Durchführung des erfindungsgemäßen Verfahrens (e) kommen alle für derartige Alkohol-Oxidationsreaktionen üblichen Oxidationsmittel infrage. Mit besonderem Vorzug verwendet man Dimethylsulfoxid in Gegenwart von geeigneten Hilfsreagenzien wie beispielsweise Oxalylchlorid in Gegenwart von Triethylamin oder Acetanhydrid, oder Schwefeltrioxid in Gegenwart von Pyridin und Triethylamin, oder p-Toluolsulfonsäurechlorid oder Sulfonsäureanhydride, wie Methansulfonsäureanhydrid oder Trifluormethansulfonsäureanhydrid oder Cyanursäurehalogenide oder Chlor oder Quecksilberacetat oder Silbertetrafluoroborat in Gegenwart von Triethylamin oder Kaliumiodid in Gegenwart von Natriumhydrogencarbonat, welche das bei der Reaktion freiwerdende Wasser binden.

Als Verdünnungsmittel zur Durchführung des erfindungsgemäßen Verfahrens (e) kommen inerte organische Lösungsmittel infrage. Vorzugsweise verwendbar sind aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid, welches gleichzeitig als Oxidationsmittel und als Verdünnungsmittel eingesetzt werden kann.

Die Reaktionstemperaturen können bei der Durchführung des erfindungsgemäßen Verfahrens (e) in einem größeren Bereich variiert werden. Im allgemeinen arbeitet man bei Temperaturen zwischen - 80 °C und + 50 °C, vorzugsweise bei Temperaturen zwischen - 80 °C und 0 °C.

Zur Durchführung des erfindungsgemäßen Verfahrens (e) setzt man pro Mol an substituiertem Triazol der Formel (Ie) im allgemeinen 1,0 bis 30,0 Mol, vorzugsweise 1,0 bis 5,0 Mol an Oxidationsmittel ein.

Die Reaktionsdurchführung, Aufarbeitung und Isolierung der Reaktionsprodukte erfolgt nach allgemein üblichen Methoden (vgl. auch die Herstellungsbeispiele).

Als Verdünnungsmittel zur Durchführung des erfindungsgemäßen Verfahrens (f) kommen inerte organische Lösungsmittel infrage. Vorzugsweise verwendbar sind aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder

Hexamethylphosphorsäuretriamid, Ester, wie Essigsäureethylester oder Sulfoxide, wie Dimethylsulfoxid, Alkohole, wie Methanol oder Ethanol oder basische Lösungsmittel wie Pyridin oder Triethylamin.

Das erfindungsgemäße Verfahren (f) wird vorzugsweise in Gegenwart eines geeigneten Reaktionshilfsmittels durchgeführt. Als solche kommen alle üblicherweise verwendbaren anorganischen und organischen Basen in Frage. Vorzugsweise verwendet man Alkalimetallhydride, -hydroxide, -alkoholate, -carbonate oder -hydrogencarbonate, wie beispielsweise Natriumhydroxid, Natriummethylat, Natriumethylat, Kalium-t-butylat, Natriumcarbonat, Kaliumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU). Auch saure Reaktionshilfsmittel wie beispielsweise p-Toluolsulfonsäure sind gegebenenfalls von Vorteil.

Die Reaktionstemperaturen können bei der Durchführung des erfindungsgemäßen Verfahrens (f) in einem größeren Bereich variiert werden. Im allgemeinen arbeitet man bei Temperaturen zwischen - 50 °C und + 150 °C, vorzugsweise bei Temperaturen zwischen 0 °C und 50 °C.

Zur Durchführung des erfindungsgemäßen Verfahrens (f) setzt man pro Mol an substituiertem Triazol der Formel (If) im allgemeinen 1,0 bis 2,0 Mol, vorzugsweise 1,0 bis 1,2 Mol an Hydroxylamin-Derivat der Formel (V) bzw. an einem entsprechenden Säureadditionssalz und gegebenenfalls 0,01 bis 20,0 Mol, vorzugsweise 0,1 bis 3,0 Mol an Reaktionshilfsmittel ein.
Die Reaktionsdurchführung, Aufarbeitung und Isolierung der Reaktionsprodukte erfolgt nach allgemein üblichen Methoden (vgl. auch die Herstellungsbeispiele).

Als Verdünnungsmittel zur Durchführung des erfindungsgemäßen Verfahrens (g) kommen inerte organische Lösungsmittel infrage. Vorzugsweise verwendbar sind aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethyl ether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (g) kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}/C_{15}$-alkylammoniumchlorid, Dibenzyl-dimethylammoniummethylsulfat, Dimethyl-$C_{12}/C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid.

Das erfindungsgemäße Verfahren (g) wird vorzugsweise in Gegenwart eines geeigneten Reaktionshilfsmittels durchgeführt. Als solche kommen alle ublicherweise verwendbaren anorganischen und organischen Basen in Frage. Vorzugsweise verwendet man Alkalimetallhydride, -hydroxide, -alkoholate, -carbonate oder -hydrogencarbonate, wie beispielsweise Natriumhydrid, Natriumhydroxid, Natriummethylat, Natriumethylat, Kalium-t-butylat, Natriumcarbonat, Kaliumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (g) in einem größeren Bereich variiert werden. Im allgemeinen arbeitet man bei Temperaturen zwischen 0 °C und 120 °C, vorzugsweise bei Temperaturen zwischen 20 °C und 100 °C.

Zur Durchführung des erfindungsgemäßen Verfahrens (g) setzt man pro Mol an substituiertem Triazol der Formel (Ii) im allgemeinen 1,0 bis 10,0 Mol, vorzugsweise 1,0 bis 3,0 Mol an einer Verbindung der Formel (VI) und gegebenenfalls 1,0 bis 5,0 Mol, vorzugsweise 1,0 bis 3,0 Mol an Reaktionshilfsmittel ein.
Die Reaktionsdurchführung, Aufarbeitung und Isolierung der Reaktionsprodukte erfolgt nach allgemein üblichen Methoden.

Als Verdünnungsmittel zur Durchführung des erfindungsgemäßen Verfahrens (h) kommen inerte organische Lösungsmittel infrage. Vorzugsweise verwendbar sind aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff oder Ether, wie Diethylether, Dioxan, Tetrahydrofuran oder Ethylenglykoldimethyl- oder -diethylether.

Das erfindungsgmäße Verfahren (h) wird vorzugsweise in Gegenwart eines geeigneten Reaktionshilfsmittels durchgeführt. Als solche kommen vorzugsweise alle üblicherweise verwendbaren anorganischen oder organischen Säuren oder andere übliche Katalysatoren infrage.
Mit besonderm Vorzug verwendet man verdünnte wässrige oder konzentrierte Mineralsäuren wie Salzsäure,

Schwefelsäure oder Phosphorsäure oder organische Sulfonsäuren wie Methansulfonsäure oder p-Toluolsulfonsäure.

Die Reaktionstemperaturen können bei der Durchführung des erfindungsgemäßen Verfahrens (h) in einem größeren Bereich variiert werden. Im allgemeinen arbeitet man bei Temperaturen zwischen 0 °C und 200 °C, vorzugsweise bei Temperaturen zwischen 20 °C und 150 °C.

Zur Durchführung des erfindungsgemäßen Verfahrens (h) setzt man pro Mol an substituiertem Triazol der Formel (If) im allgemeinen 1,0 bis 30,0 Mol, vorzugsweise 1,0 bis 5,0 Mol an Alkohol oder Diol der Formeln (VII), (VIII) oder (IX) und gegebenenfalls 0,01 bis 2,0 Mol, vorzugsweise 0,1 bis 1,0 Mol an Reaktionshilfsmittel ein.

Die Reaktionsdurchführung, Aufarbeitung und Isolierung der Reaktionsprodukte erfolgt nach allgemein üblichen Methoden (vgl. auch die Herstellungsbeispiele).

Die Säureadditionssalze der Verbindungen der Formel (I) können in einfacher Weise nach üblichen Salzbildungsmethoden, wie z.B. durch Lösen einer Verbindung der Formel (I) in einem geeigneten inerten Lösungsmittel und Hinzufügen der Säure, wie z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die Metallsalzkomplexe von Verbindungen der Formel (I) können in einfacher Weise nach üblichen Verfahren erhalten werden, so z.B. durch Lösen des Metallsalzes in Alkohol, z.B. Ethanol und Hinzufügen zur Verbindung der Formel (I). Man kann Metallsalz-Komplexe in bekannter Weise, z.B. durch Abfiltrieren isolieren und gegebenenfalls durch Umkristallistion reinigen.

Die erfindungsgemäßen Wirkstoffe weisen eine stark mikrobizide Wirkung auf und können zur Bekämpfung von unerwünschten Mikroorganismen als Fungizide und Bakterizide praktisch eingesetzt werden.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen den Erreger der Braunfleckigkeit des Weizens (Leptosphaeria nodorum) oder gegen den Erreger der Braunfleckigkeit der Gerste (Pyrenophora teres) oder gegen den Erreger der Blattfleckenkrankheit des Weizens (Cochliobolus sativus) oder gegen den Erreger des echten Getreidemehltaus (Erysiphe graminis) oder gegen den Erreger des Getreideschneeschimmels (Fusarium nivale) oder gegen den Erreger der Getreidestengelgrundfäule (Fusarium culmorum) sowie zur Bekämpfung von Krankheiten im Obst- und Gemüseanbau, wie beispielsweise gegen den Erreger des Apfelschorfes (Venturia inaequalis) oder gegen den Erreger des Gurkenmehltaus (Sphaerotheca fuliginea) oder zur Bekämpfung von Reiskrankheiten, wie beispielsweise gegen den Erreger der Reisfleckenkrankheit (Pyricularia oryzae) eingesetzt werden.

Außerdem besitzen die erfindungsgemäßen Wirkstoffe auch pflanzenwachstumsregulierende Wirksamkeit.

Schließlich lassen sich die erfindungsgemäßen Wirkstoffe im Materialschutz zum Schutz von technischen Materialien gegen Befall durch Mikroorganismen einsetzen. Unter technischen Materialien sind in diesem Zusammenhang nicht lebende Materialien zu verstehen, die für die Verwendung in der Technik zubereitet worden sind. Beispielsweise können technische Materialien, die durch die erfindungsgemäßen Wirkstoffe vor mikrobieller Veränderung oder Zerstörung geschützt werden sollen, Klebstoffe, Leime, Papier, Karton, Textilien, Leder, Holz, Anstrichmittel, Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Kühlkreisläufe genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten), sowie gegen Schleimorganismen und Algen.

Es seien beispielsweise Mikroorganismen der folgenden Gattungen genannt:

Alternaria, wie Alternaria tenuis,
Aspergillus, wie Aspergillus niger,
Chaetomium, wie Chaetomium globosum,
Coniophora, wie Coniophora puteana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage; Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse

Kieselsäure, Aluminiumoxid und Silikate; als feste Trägerstoffe für Granulate kommen in Frage; z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage; z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellung und Verwendung der erfindungsgemäßen Wirkstoffe werden durch die nachfolgenden Beispiele veranschaulicht.

Herstellungsbeispiele

Beispiel 1

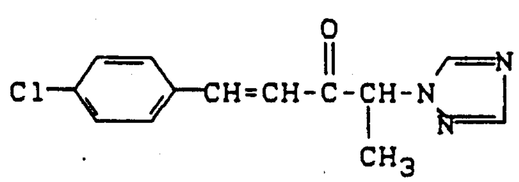

(Verfahren a)

Zu 123,4 g (0,878 Mol) 4-Chlorbenzaldehyd und 122,6 g (0,881 Mol) 3-(1,2,4-Triazol-1-yl)-butan-2-on in 430 ml Chloroform gibt man 3,8 ml (0,044 Mol) Piperidin und 9,8 ml (0.171 Mol) Essigsäure und kocht die Mischung 26 Stunden unter Rückfluß über einem Wasserabscheider. Zur Aufarbeitung wäscht man die erkaltete Reaktionsmischung nacheinander mit 500 ml Wasser, 200 ml 40prozentiger wässriger Natriumhydrogensulfitlösung und nochmal mit 500 ml Wasser. Man trocknet die organische Phase über Natriumsulfat, engt unter vermindertem Druck ein, nimmt den Rückstand in 200 ml heißem Essigester auf und fällt durch Zusatz von 400 ml Petrolether das gewünschte Produkt aus.

Nach Absaugen und Trocknen erhält man 117 g (51 % der Theorie) an 1-(4-Chlorphenyl)-4-(1,2,4-triazol-1-yl)-pent-1-en-3-on vom Schmelzpunkt 115 °C - 116 °C.

Beispiel 2

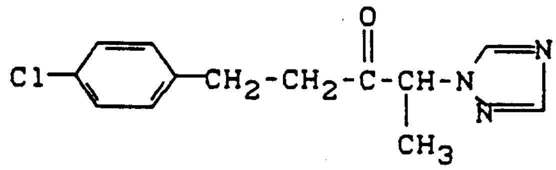

(Verfahren b)

Zu einer Lösung von 35 g (0,14 Mol) 1-(4-Chlorphenyl)-4-(1,2,4-triazol-1-yl)-pent-1-en-3-on in 180 ml Methanol gibt man 5 g Raney-Nickel und hydriert anschließend unter Rühren 2 Stunden bei 60 °C bis 70 °C und 90 bis 100 bar Wasserstoffdruck. Zur Aufarbeitung wird das Reaktionsgemisch filtriert, das Filtrat eingeengt und der Rückstand zweimal aus Diisopropylether umkristallisiert.

Man erhält 24,9 g (71 % der Theorie) an 1-(4-Chlorphenyl)-4-(1,2,4-triazol-1-yl)-pentan-3-on vom Schmelzpunkt 77 °C - 78 °C.

Beispiel 3

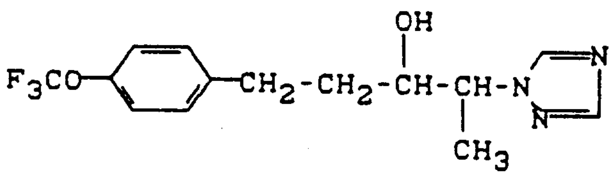

(Verfahren b)

Zu einer Lösung von 250 g (0,8 Mol) 1-(4-Trifluormethoxyphenyl)-4-(1,2,4-triazol-1-yl)-pent-1-en-3-on in 1.500 ml Methanol gibt man 40 g Raney-Nickel und hydriert anschließend unter Rühren 6,5 Stunden bei 90 °C bis 110 °C und 90 bis 100 bar Wasserstoffdruck. Zur Aufarbeitung wird das Reaktionsgemisch filtriert, das Filtrat eingeengt und der Rückstand durch Verrühren mit Diisopropylether zur Kristallisation gebracht.

Man erhält 148 g (59 % der Theorie) an 1-(4-Trifluormethoxyphenyl)-4-(1,2,4-triazol-1-yl)-pentan-3-ol vom Schmelzpunkt 69 °C.

Beispiel 4

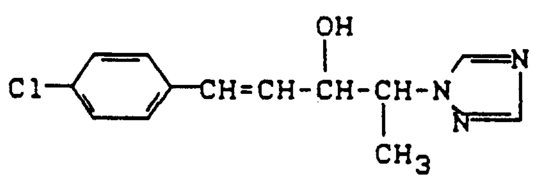

(Verfahren c)

Zu einer Suspension aus 12,4 g (0,0474 Mol) 1-(4-Chlorphenyl)-4-(1,2,4-triazol-1-yl)-pent-1-en-3-on und 3,53 g (0,0318 Mol) wasserfreiem Calciumchlorid in 100 ml Isopropanol tropft man bei - 5 °C bis 0 °C

innerhalb von 30 Minuten unter Rühren eine Lösung von 1,26 g (0,0333 Mol) Natriumborhydrid in 15 ml Wasser und rührt nach beendeter Zugabe 2 Stunden bei Raumtemperatur. Zur Aufarbeitung wird die Reaktionsmischung unter vermindertem Druck eingeengt und der verbleibende Rückstand in eine Mischung aus 150 ml Wasser und 10 ml Essigsäure gegeben. Es wird mehrfach mit jeweils 50 ml Dichlormethan extrahiert. Die vereinigten organischen Phasen werden mit Wasser gewaschen, über Natriumsulfat getrocknet, unter vermindertem Druck eingeengt, und der Rückstand aus 30 ml Acetonitril umkristallisiert.

Man erhält 8,9 g (72 % der Theorie) an 1-(4-Chlorphenyl)-4-(1,2,4-triazol-1-yl)-pent-1-en-3-ol vom Schmelzpunkt 117 °C - 118 °C.

Beispiel 5

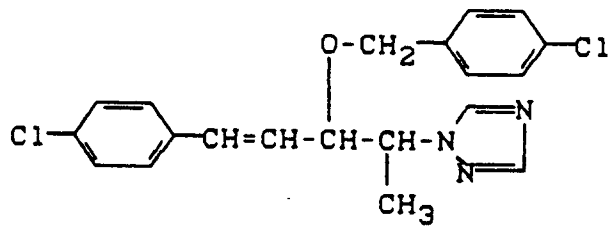

(Verfahren d)

Zu einer Suspension von 0,36 g (0,012 Mol) 80prozentigem Natriumhydrid (in Paraffin) in 20 ml absolutem Dimethoxyethan gibt man 0,2 g (0,0012 Mol) Kaliumiodid und tropft anschließend bei 0 °C unter Rühren eine Lösung von 2,6 g (0,010 Mol) 1-(4-Chlorphenyl)-4-(1,2,4-triazol-1-yl)-pent-1-en-3-ol in 10 ml Dimethoxyethan zu. Man wartet das Ende der Gasentwicklung ab, gibt dann 1,6 g (0,010 Mol) 4-Chlorbenzylchlorid in 5 ml Dimethoxyethan zu, rührt 4 Stunden bei Raumtemperatur und anschließend 20 Stunden bei 40 °C. Zur abgekühlten Reaktionsmischung gibt man 10 ml Isopropanol, gießt dann die Mischung in Eiswasser, extrahiert dreimal mit Dichlormethan, trocknet die organische Phase über Natriumsulfat, engt unter vermindertem Druck ein und kristallisiert den Rückstand durch Behandeln mit einer Mischung aus Diethylether/n-Hexan/Ethanol = (5:3:1).

Man erhält 0,6 g (16 % der Theorie) an 1-(4-Chlorphenyl)-3-(4-Chlorbenzyloxy)-4-(1,2,4-triazol-1-yl)-pent-1-en vom Schmelzpunkt 91 °C.

Beispiel 6

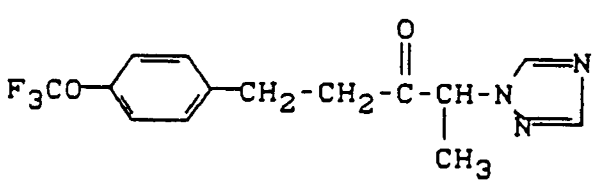

(Verfahren e)

Zu einer Lösung von 7,0 g (0,055 Mol) Oxalylchlorid in 25 ml absolutem Dichlormethan tropft man bei - 50 °C innerhalb von 10 Minuten 9,4 g (0,12 Mol) Dimethylsulfoxid in 10 ml Dichlormethan. Dann gibt man ebenfalls bei - 50 °C eine Suspension von 15,8 g (0,05 Mol) 1-(4-Trifluormethoxyphenyl)-4-(1,2,4-triazol-1-yl)-pentan-3-ol in 25 ml Dichlormethan zu, rührt 20 Minuten bei - 50 °C, gibt dann 25,3 g (0,25 Mol) Triethylamin zu und rührt weitere 10 Minuten bei - 50 °C. Zur Aufarbeitung läßt man die Temperatur der Reaktionsmischung auf 0 °C ansteigen, gibt 20 ml Wasser zu und trennt die organische Phase ab. Man trocknet die organische Phase über Magnesiumsulfat und entfernt das Lösungsmittel unter vermindertem Druck (0,05 mbar/50 °C).

Man erhält 15 g (96 % der Theorie) an 1-(4-Trifluormethoxyphenyl)-4-(1,2,4-triazol-1-yl)-pentan-3-on vom Schmelzpunkt 58 °C.

Beispiel 7

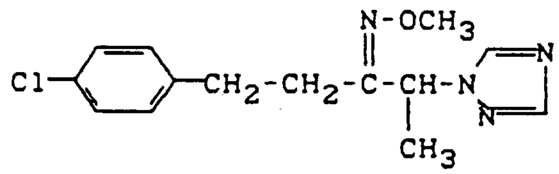

(Verfahren f)

Zu 10,0 g (0,0379 Mol) 1-(4-Chlorphenyl)-4-(1,2,4-triazol-1-yl)-pentan-3-on in 75 ml trockenem Pyridin gibt man 3,17 g (0,0379 Mol) O-Methylhydroxylaminhydrochlorid. Man rührt 20 Stunden bei Raumtemperatur, engt dann das Reaktionsgemisch unter vermindertem Druck ein, nimmt den Rückstand in Dichlormethan auf, wäscht mit Wasser, trocknet die organische Phase über Natriumsulfat, engt wiederum unter vermindertem Druck ein und chromatographiert den Rückstand an Kieselgel (Laufmittel:Essigester).

Man erhält 7,2 g (65 % der Theorie) 1-(4-Chlorphenyl)-3-methoximino-4-(1,2,4-triazol-1-yl)-pentan als Öl vom Brechungsindex $n_D^{20}$ 1,5448.

Beispiel 8

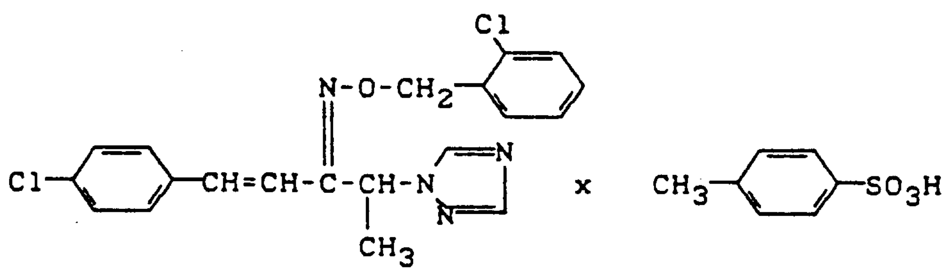

(Verfahren f)

3,2 g (0,02 Mol) 0-(2-Chlorbenzyl)-hydroxylamin und 5,2 g (0,02 Mol) 1-(4-Chlorphenyl)-4-(1,2,4-triazol-1-yl)-pent-1-en-3-on werden zusammen mit 2,0 g (0,012 Mol) p-Toluolsulfonsäure in 160 ml Toluol 18 Stunden bei Rückflußtemperatur über einem Wasserabscheider gekocht. Man filtriert das aus der erkalteten Reaktionsmischung ausgefallene Salz ab, verrührt kurzzeitig mit gesättigter wässriger Natriumhydrogencarbonatlösung, filtriert und verreibt den Rückstand mit Diethylether.

Man erhält 5,7 g (50 % der Theorie) an 1-(4-Chlorphenyl)-3-(2-chlorbenzyloximino)-4-(1,2,4-triazol-1-yl)-pent-1-en p-Toluolsulfonsäuresalz vom Schmelzpunkt 167 °C.

Beispiel 9

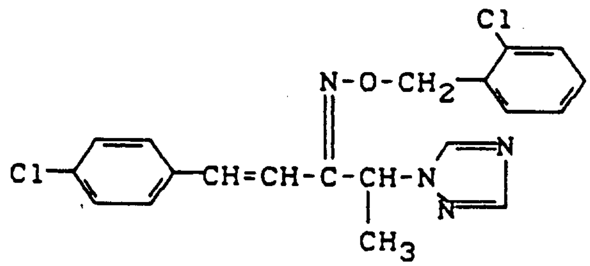

5,5 g (0,0096 Mol) 1-(4-Chlorphenyl)-3-(2-chlorbenzyloximino)-4-(1,2,4-triazol-1-yl)-pent-1-en p-Toluolsulfonsäuresalz werden zusammen mit 2,0 g (0,02 Mol) Triethylamin in 20 ml Dichlormethan 10 Minuten bei Raumtemperatur gerührt. Anschließend wäscht man das Reaktionsgemisch mit 10 ml Wasser, trocknet die organische Phase über Natriumsulfat und entfernt das Lösungsmittel unter vermindertem Druck.

Man erhält 3,6 g (99 % der Theorie) an 1-(4-Chlorphenyl)-3-(2-chlorbenzyloximino)-4-(1,2,4-triazol-1-yl)-pent-1-en als Öl vom Brechungsindex $n_D^{20}$ 1,6101.

Beispiel 10

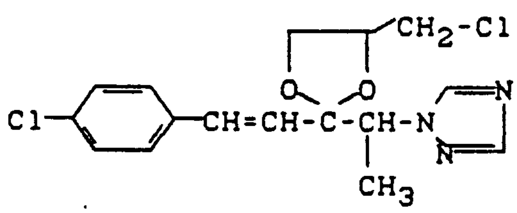

(Verfahren h)

3,5 g (0,013 Mol) 1-(4-Chlorphenyl)-4-(1,2,4-triazol-1-yl)-pent-1-en-3-on und 1,77 g (0,016 Mol) 3-Chlorpropan-1,2-diol werden mit 0,1 ml Methansulfonsäure in 100 ml absolutem Toluol 82 Stunden auf Rückflußtemperatur erhitzt. Anschließend wird das Reaktionsgemisch 3-mal mit je 100 ml 0,1 n wäßriger Natronlauge und dann 3-mal mit je 50 ml Wasser gewaschen. Die organische Phase wird über Kaliumcarbonat getrocknet, das Lösungsmittel wird abdestilliert und der Rückstand über Kieselgel chromatographiert (Laufmittel: Essigester).

Man erhält 1,45 g (31 % der Theorie) an 2-[1-(1,2,4-Triazol-1-yl )ethyl]-2-[2-(4-chlorphenyl)-ethenyl]-4-chlormethyl-1,3-dioxolan als Öl vom Brechungsindex $n_D^{20}$ = 1,5591.

In entsprechender Weise und gemäß den allgemeinen Angaben zur Herstellung erhält man die substituierten Triazole der Formel

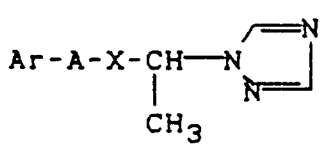

(I),

die in der folgenden Tabelle 1 formelmäßig aufgeführt sind.

Tabelle 1

| Bsp. Nr. | Ar | A | X | physikalische Eigenschaften |
|---|---|---|---|---|
| 11 | 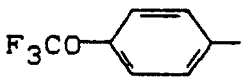 | -CH=CH- | 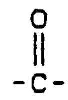 | Fp 80-82 °C |
| 12 | 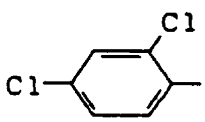 | -CH=CH- | 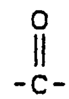 | Fp 104-106 °C |
| 13 | 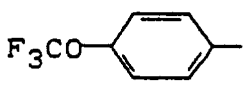 | -CH=CH- | 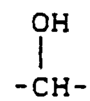 | $n_D^{20}$ 1,5159 |
| 14 | 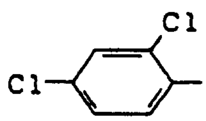 | -CH=CH- | 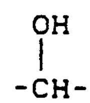 | Fp 124-126 °C |

Tabelle 1 (Fortsetzung)

| Bsp. Nr. | Ar | A | X | physikalische Eigenschaften |
|---|---|---|---|---|
| 15 | 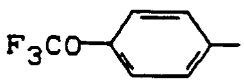 | 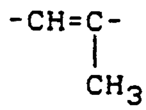 | 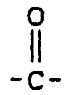 | $n_D^{20}$ 1,5310 |
| 16 | 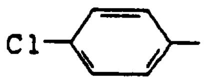 | 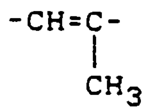 | 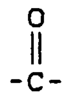 | Fp 56-58 °C |
| 17 | 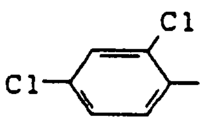 | 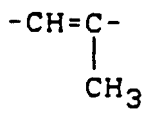 | 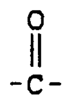 | Fp 114-116 °C |
| 18 | 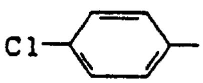 | -CH=CH- | 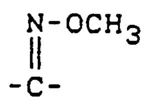 | Fp 102-104 °C |
| 19 | 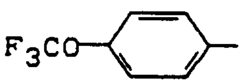 | 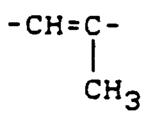 | 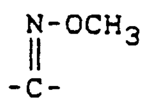 | $n_D^{20}$ 1,5068 |
| 20 | 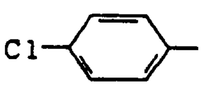 | 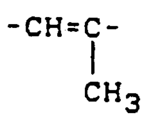 | 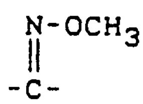 | $n_D^{20}$ 1,5770 |

Tabelle 1 (Fortsetzung)

| Bsp. Nr. | Ar | A | X | physikalische Eigenschaften |
|---|---|---|---|---|
| 21 | 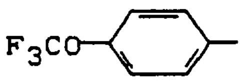 | -CH=CH- | 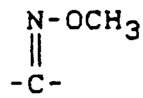 | $n_D^{20}$ 1,5400 |
| 22 | 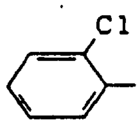 | -CH=CH- | 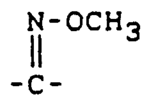 | $n_D^{20}$ 1,5408 |
| 23 | 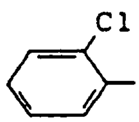 | -CH=CH- | 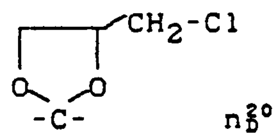 | $n_D^{20}$ 1,5543 |
| 24 | 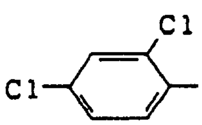 | 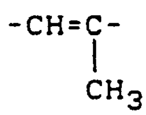 | 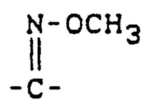 | $n_D^{20}$ 1,5766 |
| 25 | 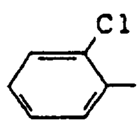 | 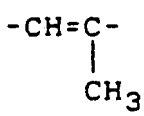 | 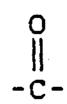 | Fp 78-80 °C |
| 26 | 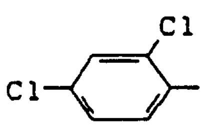 | $-CH_2-CH_2$ | 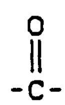 | $n_D^{20}$ 1,5546 |
| 27 | 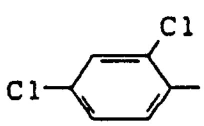 | -CH=CH- | 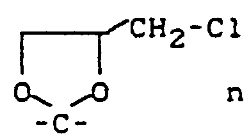 | $n_D^{20}$ 1,5515 |

Tabelle 1 (Fortsetzung)

| Bsp. Nr. | Ar | A | X | physikalische Eigenschaften |
|---|---|---|---|---|
| 28 | 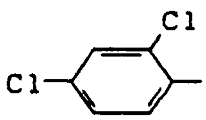 | -CH=CH- | 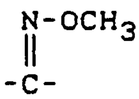 | $n_D^{20}$ 1,6095 |
| 29 | 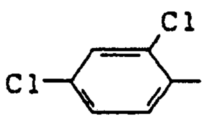 | $-CH_2-CH_2-$ | 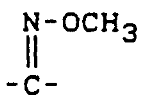 | $n_D^{20}$ 1,5520 |
| 30 | 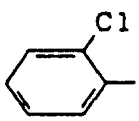 | 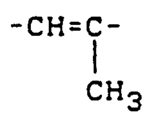 | 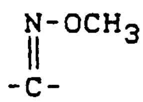 | $n_D^{20}$ 1,5720 |
| 31 | 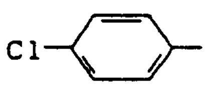 | -CH=CH- | 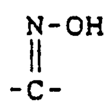 | Fp 169-170 °C |
| 32 | 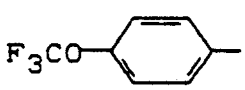 | $-CH_2-CH_2-$ | 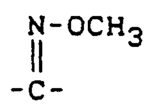 | $n_D^{20}$ 1,4918 |
| 33 | 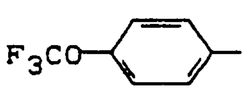 | -CH=CH- | 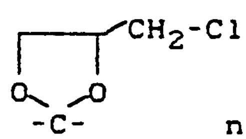 | $n_D^{20}$ 1,5180 |
| 34 | 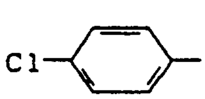 | -CH=CH- | 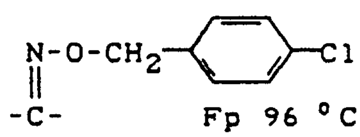 | Fp 96 °C |

Tabelle 1 (Fortsetzung)

| Bsp. Nr. | Ar | A | X | physikalische Eigenschaften |
|---|---|---|---|---|
| 35 | 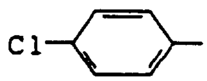 | -CH=CH- | 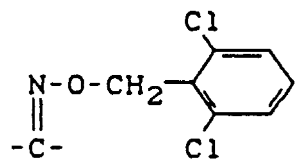 | $n_D^{20}$ 1,5180 |
| 36 | 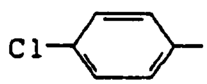 | -CH=CH- | 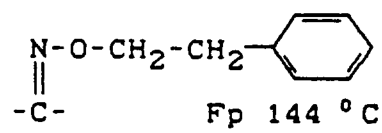 | Fp 144 °C |
| 37 | 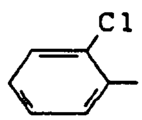 | -CH=CH- | 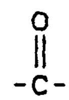 | $n_D^{20}$ 1,5810 |
| 38 | 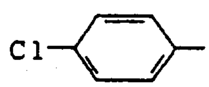 | 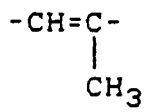 | 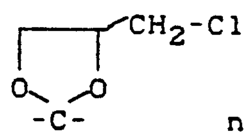 | $n_D^{20}$ 1,5522 |
| 39 | 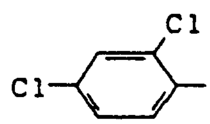 | $-CH_2-CH_2-$ | 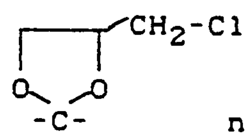 | $n_D^{20}$ 1,5578 |
| 40 | 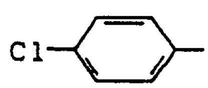 | $-CH_2-CH_2-$ | 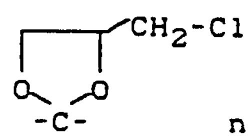 | $n_D^{20}$ 1,5492 |

Tabelle 1 (Fortsetzung)

| Bsp. Nr. | Ar | A | X | physikalische Eigenschaften |
|---|---|---|---|---|
| 41 | 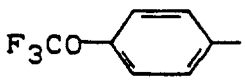 | $-CH_2-CH_2-$ | 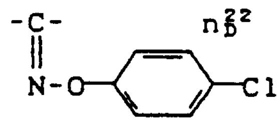 | $n_D^{22}$ 1,5296 |
| 42 | 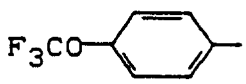 | $-CH_2-CH_2-$ | 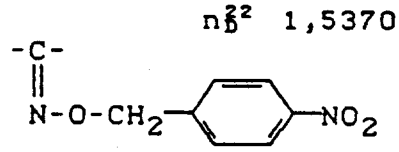 | $n_D^{22}$ 1,5370 |
| 43 | 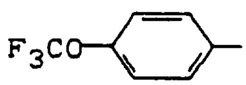 | $-CH_2-CH_2-$ | 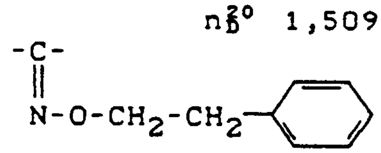 | $n_D^{20}$ 1,5092 |
| 44 | 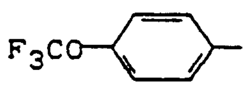 | $-CH_2-CH_2-$ | 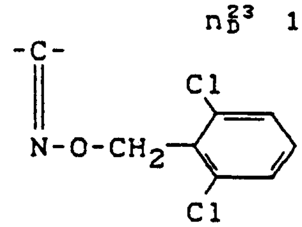 | $n_D^{23}$ 1,5110 |
| 45 | 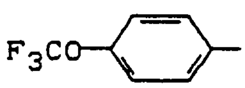 | $-CH_2-CH_2-$ | 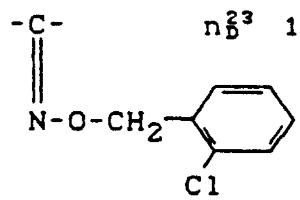 | $n_D^{23}$ 1,5217 |

Tabelle 1 (Fortsetzung)

| Bsp. Nr. | Ar | A | X | physikalische Eigenschaften |
|---|---|---|---|---|
| 46 | 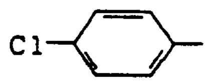 | $-CH_2-CH_2-$ | 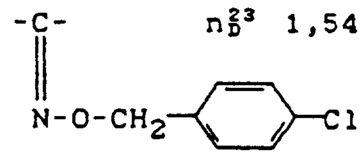 | $n_D^{23}$ 1,5498 |
| 47 | 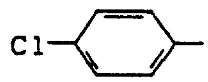 | $-CH_2-CH_2-$ | 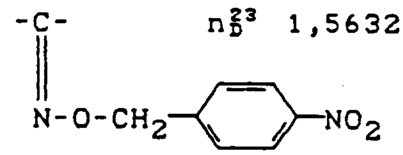 | $n_D^{23}$ 1,5632 |
| 48 | 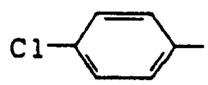 | $-CH_2-CH_2-$ | 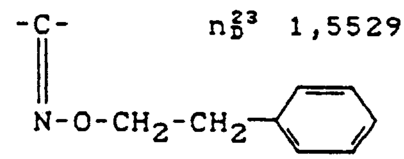 | $n_D^{23}$ 1,5529 |
| 49 | 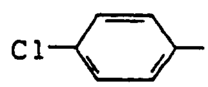 | $-CH_2-CH_2-$ | 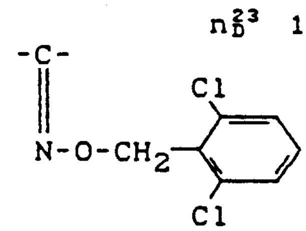 | $n_D^{23}$ 1,5731 |
| 50 | 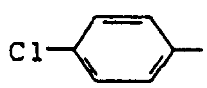 | $-CH_2-CH_2-$ | 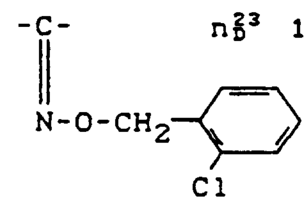 | $n_D^{23}$ 1,5575 |

Tabelle 1 (Fortsetzung)

| Bsp. Nr. | Ar | A | X | physikalische Eigenschaften |
|---|---|---|---|---|
| 51 | 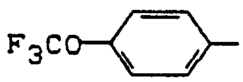 | $-CH_2-CH_2$ | 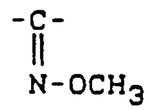 | Fp 124 °C |
| 52 | 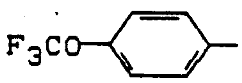 | $-CH_2-CH_2-$ | 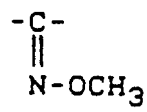 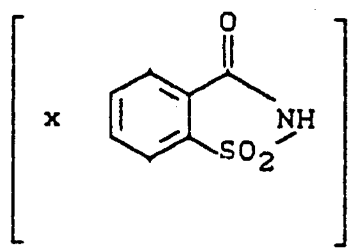 | Fp 224 °C |
| 53 | 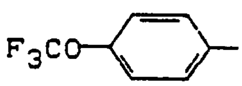 | $-CH_2-CH_2-$ | 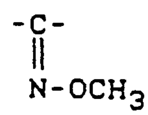 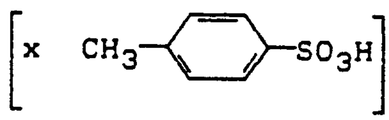 | Fp 110 °C |

Anwendungsbeispiele

In den folgenden Anwendungsbeispielen wurden die nachstehend aufgeführten Verbindungen als Vergleichssubstanzen eingesetzt:

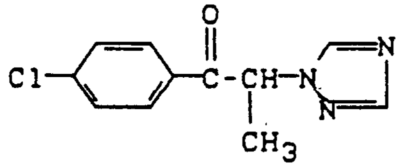 (A)

1-(4-Chlorphenyl)-2-(1,2,4-triazol-1-yl)-propan-1-on

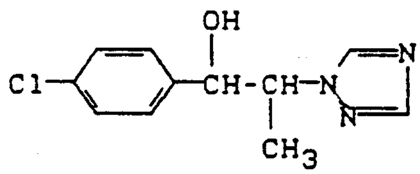 (B)

1-(4-Chlorphenyl)-2-(1,2,4-triazol-1-yl)-propan-1-ol
(beide bekannt aus DE-A 24 31 407)

Beispiel A

Pyrenophora teres-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Abtrocknen des Spritzbelages werden die Pflanzen mit einer Konidiensuspension von Pyrenophora teres besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

In diesem Test zeigen die in den Beispielen 7, 19, 21, 29, 32 und 33 aufgeführten erfindungsgemäßen Stoffe eine wesentlich bessere Wirksamkeit als die Vergleichssubstanzen (A) und (B).

Beispiel B

Leptosphaeria nodorum-Test (Weizen)/protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 15 °C und einer relativen Luftfeuchtigkeit von ca. 80 % aufgestellt.

10 Tage nach der Inokulation erfolgt die Auswertung.

In diesem Test zeigen die in den Beispielen 7, 19, 20, 21, 29, 32 und 33 aufgeführten erfindungsgemäßen Stoffe eine wesentlich bessere Wirksamkeit als die Vergleichssubstanzen (A) und (B).

Beispiel C

Zum Nachweis der Wirksamkeit gegen Pilze, die im Materialschutz zu bekämpfen sind, werden die minimalen Hemm-Konzentrationen (MHK) von erfindungsgemäßen Wirkstoffen bestimmt.

Ein Agar, der aus Bierwürze und Pepton hergestellt wird, wird mit erfindungsgemäßen Wirkstoffen in Konzentrationen von 0,1 mg/l bis 5000 mg/l versetzt. Nach Erstarren des Agars erfolgt Kontamination mit Reinkulturen der in der Tabelle C aufgeführten Testorganismen. Nach 2-wöchiger Lagerung bei 28 °C und

60 bis 70 % rel. Luftfeuchtigkeit wird die MHK bestimmt. MHK ist die niedrigste Konzentration an Wirkstoff, bei der keinerlei Bewuchs durch die verwendete Mikrobenart erfolgt.

In diesem Test zeigen die in den Beispielen 1, 7 und 12 aufgeführten erfindungsgemäßen Wirkstoffe bei Aspergillus niger, Chaetomium globosum und Penicillium glaucum MHK-Werte zwischen 50 und 1000 mg/l.

**Patentansprüche**

**1.** Substituierte Triazole der Formel

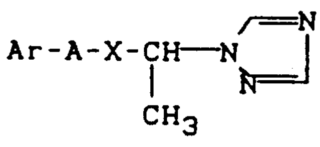

(I)

in welcher

Ar für Aryl mit 6 bis 10 Kohlenstoffatomen steht, das einfach oder mehrfach, gleichartig oder verschieden substituiert sein kann durch Halogen, Cyano, Nitro, jeweils geradkettiges oder verzweigtes Alkyl, Alkoxy und Alkylthio mit jeweils 1 bis 4 Kohlenstoffatomen, jeweils geradkettiges oder verzweigtes Halogenalkyl, Halogenalkoxy und Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl und Alkoximinoalkyl mit jeweils 1 bis 4 Kohlenstoffatomen im Alkoxyteil und 1 bis 4 Kohlenstoffatomen im Alkylteil, gegebenenfalls einfach oder mehrfach, gleichartig oder verschieden durch Halogen und/oder geradkettiges oder verzweigtes Alkyl mit 1 bis 4 Kohlenstoffatomen substituiertes Phenyl, gegebenenfalls einfach oder mehrfach, gleichartig oder verschieden durch Halogen und/oder geradkettiges oder verzweigtes Alkyl mit 1 bis 4 Kohlenstoffatomen substituiertes Phenoxy und/oder durch gegebenenfalls einfach oder mehrfach, gleichartig oder verschieden durch Halogen und/oder geradkettiges oder verzweigtes Alkyl mit 1 bis 4 Kohlenstoffatomen substituiertes Benzyloxy,

A für die Gruppe

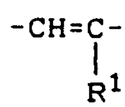 oder 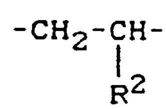

steht und

X für die Gruppen

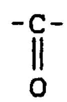 ; 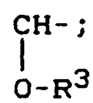 ; 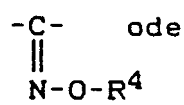 oder

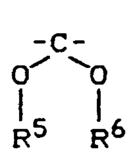

steht,

wobei

$R^1$ für Wasserstoff oder für geradkettiges oder verzweigtes Alkyl mit 1 bis 4 Kohlenstoffatomen steht,

$R^2$ für Wasserstoff oder für geradkettiges oder verzweigtes Alkyl mit 1 bis 4 Kohlenstoffatomen steht,

$R^3$ Wasserstoff, für jeweils geradkettiges oder verzweigtes Alkyl mit 1 bis 8 Kohlenstoffatomen, Alkenyl mit 3 bis 8 Kohlenstoffatomen oder Alkanoyl mit 1 bis 6 Kohlenstoffatomen im Alkanteil steht, außerdem für Cycloalkyl mit 3 bis 7 Kohlenstoffatomen steht, das einfach oder mehrfach, gleichartig oder verschieden substituiert sein kann durch Halogen, geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen, oder für Aralkyl mit 6 bis 10 Kohlenstoffatomen im Arylteil und 1 bis 4 Kohlenstoffatomen im geradkettigen oder verzweigten Alkylteil steht, wobei jeder der Reste einfach oder mehrfach, gleichartig oder verschieden im Arylteil substituiert sein kann durch Halogen, Cyano, Nitro, jeweils geradkettiges oder verzweigtes Alkyl, Alkoxy oder Alkylthio mit jeweils 1 bis 4 Kohlenstoffatomen und/oder jeweils geradkettiges oder verzweigtes Halogenalkyl, Halogenalkoxy

oder Halogenalkylthio mit jeweils 1 bis 4 Kohlenstoffatomen und 1 bis 9 gleichen oder verschiedenen Halogenatomen, oder für Aroyl mit 6 bis 10 Kohlenstoffatomen im Arylteil steht, wobei jeder dieser Arylreste einfach oder mehrfach, gleichartig oder verschieden substituiert sein kann durch Halogen, Cyano, Nitro, jeweils geradkettiges oder verzweigtes Alkyl, Alkoxy oder Alkylthio mit jeweils 1 bis 4 Kohlenstoffatomen und/oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen,

$R^4$ für Wasserstoff, geradkettiges oder verzweigtes Alkyl mit 1 bis 6 Kohlenstoffatomen oder für Cycloalkyl mit 3 bis 7 Kohlenstoffatomen steht, wobei jeder der Cycloalkylreste einfach oder mehrfach, gleichartig oder verschieden substituiert sein kann durch Halogen, geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen, oder für Aralkyl mit 6 bis 10 Kohlenstoffatomen im Arylteil und 1 bis 4 Kohlenstoffatomen im geradkettigen oder verzweigten Alkylteil steht, wobei jeder der Arylreste einfach oder mehrfach, gleichartig oder verschieden substituiert sein kann durch

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy und/oder Halogenalkylthio mit jeweisl 1 bis 4 Kohlenstoffatomen und 1 bis 9 gleichen oder verschiedenen Halogenatomen, oder für Aryl mit 6 bis 10 Kohlenstoffatomen steht, wobei jeder der Arylreste einfach oder mehrfach, gleichartig oder verschieden substituiert sein kann durch Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy und/oder Halogenalkylthio mit jeweils 1 bis 4 Kohlenstoffatomen und 1 bis 9 gleichen oder verschiedenen Halogenatomen und

$R^5$ und $R^6$ unabhängig voneinander für Alkyl mit 1 bis 6 Kohlenstoffatomen oder Aralkyl mit 6 bis 10 Kohlenstoffatomen im Arylteil und 1 bis 4 Kohlenstoffatomen im geradkettigen oder verzweigten Alkylteil stehen, wobei jeder der Arylreste einfach oder mehrfach, gleichartig oder verschieden substituiert sein kann durch Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy und/oder Halogenalkylthio mit

jeweils 1 bis 4 Kohlenstoffatomen und 1 bis 9 gleichen oder verschiedenen Halogenatomen, oder

$R^5$ und $R^6$ gemeinsam für einen Alkylenrest mit 2 bis 4 Kohlenstoffatomen stehen, wobei der Alkylenrest einfach oder mehrfach, gleichartig oder verschieden substituiert sein kann durch geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, Alkoxyalkyl mit 1 bis 4 Kohlenstoffatomen im Alkylteil und 1 bis 4 Kohlenstoffatomen im Alkoxyteil und/oder Arylalkyloxyalkyl mit 6 bis 10 Kohlenstoffatomen im Arylteil und 1 bis 4 Kohlenstoffatomen im geradkettigen oder verzweigten Alkyloxyteil sowie 1 bis 4 Kohlenstoffatomen im geradkettigen oder verzweigten Alkylteil, wobei der Arylrest einfach oder mehrfach, gleichartig oder verschieden substituiert sein kann durch Halogen und/oder Alkyl mit 1 bis 4 Kohlenstoffatomen,

sowie deren Säureadditionssalze und Metallsalzkomplexe.

**2.** Verfahren zur Herstellung von substituierten Triazolen der Formel (I) gemäß Anspruch 1 sowie von deren Säureadditions-Salzen und Metallsalz-Komplexen, dadurch gekennzeichnet, daß man

a) zur Herstellung von substituierten Triazolen der Formel

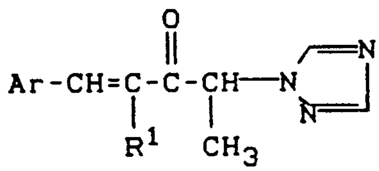

(Ia)

in welcher

Ar und $R^1$ die oben angegebene Bedeutung haben,

aromatische Aldehyde der Formel

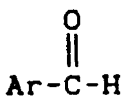

(II)

in welcher

Ar die oben angegebene Bedeutung hat,

mit Triazolylketonen der Formel

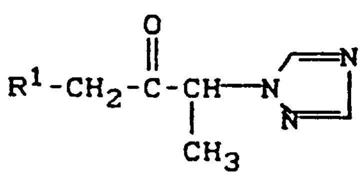

(III)

in welcher

$R^1$ die oben angegebene Bedeutung hat,

gegebenenfalls in Gegenwart eines Verdünnungsmittels und gegebenenfalls in Gegenwart eines Reaktionshilfsmittels umsetzt;

(b) zur Herstellung von substituierten Triazolen der Formel

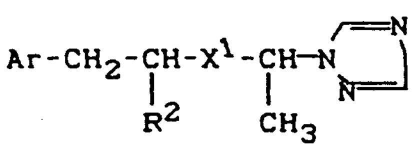

(Ib)

in welcher

$X^1$ für eine der Gruppen

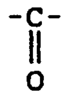 oder 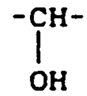

steht und

Ar und $R^2$ die oben angegebene Bedeutung haben,

substituierte Triazole der Formel

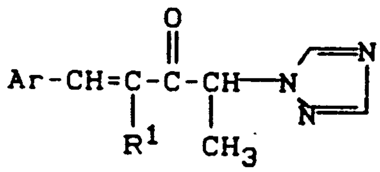 (Ia)

in welcher

Ar und $R^1$ die oben angegebene Bedeutung haben,

mit Wasserstoff in Gegenwart eines Hydrierkatalysators und in Gegenwart eines Verdünnungsmittels hydriert;

(c) zur Herstellung von substituierten Triazolen der Formel

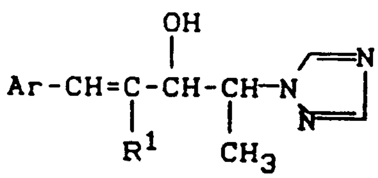 (Ic)

in welcher

Ar und $R^1$ die oben angegebene Bedeutung haben,

substituierte Triazole der Formel

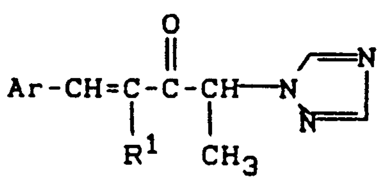 (Ia)

in welcher

Ar und $R^1$ die oben angegebene Bedeutung haben,

mit komplexen Hydriden in Gegenwart eines Verdünnungsmittels reduziert;

(d) zur Herstellung von substituierten Triazolen der Formel

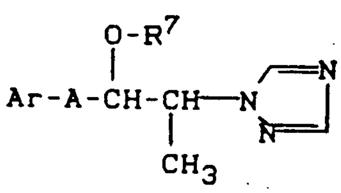 (Id)

in welcher

$R^7$ für jeweils geradkettiges oder verzweigtes Alkyl mit 1 bis 8 Kohlenstoffatomen, Alkenyl mit 3 bis 8 Kohlenstoffatomen oder Alkanoyl mit 1 bis 6 Kohlenstoffatomen

im Alkanteil steht, außerdem für Cycloalkyl mit 3 bis 7 Kohlenstoffatomen steht, das einfach oder mehrfach, gleichartig oder verschieden substituiert sein kann durch Halogen, geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen, oder für Aralkyl mit 6 bis 10 Kohlenstoffatomen im Arylteil und 1 bis 4 Kohlenstoffatomen im geradkettigen oder verzweigten Alkylteil steht, wobei jeder der Reste einfach oder mehrfach, gleichartig oder verschieden im Arylteil substituiert sein kann durch Halogen, Cyano, Nitro, jeweils geradkettiges oder verzweigtes Alkyl, Alkoxy oder Alkylthio mit jeweils 1 bis 4 Kohlenstoffatomen und/oder jeweils geradkettiges oder verzweigtes Halogenalkyl, Halogenalkoxy

oder Halogenalkylthio mit jeweils 1 bis 4 Kohlenstoffatomen und 1 bis 9 gleichen oder verschiedenen Halogenatomen, oder für Aroyl mit 6 bis 10 Kohlenstoffatomen im Arylteil steht, wobei jeder dieser Arylreste einfach oder mehrfach, gleichartig oder verschieden substituiert sein kann durch Halogen, Cyano, Nitro, jeweils geradkettiges oder verzweigtes Alkyl, Alkoxy oder Alkylthio mit jeweils 1 bis 4 Kohlenstoffatomen und/oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen,

Ar und A die oben angegebene Bedeutung haben,

substituierte Triazole der Formel

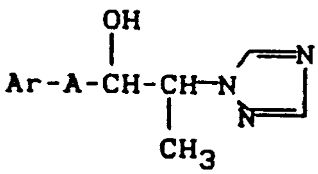

(Ie)

in welcher

Ar und A die oben angegebene Bedeutung haben,

mit Verbindungen der Formel

$R^7$-E (IV)

in welcher

$R^7$ die oben angegebene Bedeutung hat und

E für Chlor, Brom, Iod, Methylsulfonyloxy, p-Methyl-phenyl-sulfonyloxy oder einen Essigsäure- oder Propionsäure-anhydrid-Rest steht,

gegebenenfalls in Gegenwart eines Verdünnungsmittels und gegebenenfalls in Gegenwart eines Reaktionshilfsmittels umsetzt;

(e) zur Herstellung von substituierten Triazolen der Formel

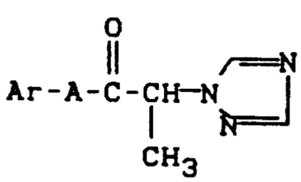

(If)

in welcher

Ar und A die oben angegebene Bedeutung haben,

substituierte Triazole der Formel

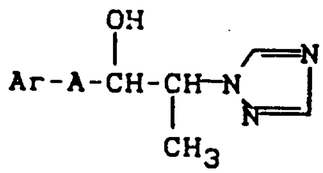

(Ie)

in welcher

Ar und A die oben angegebene Bedeutung haben,

mit einem Oxidationsmittel gegebenenfalls in Gegenwart eines Verdünnungsmittels umsetzt;

(f) zur Herstellung von substituierten Triazolen der Formel

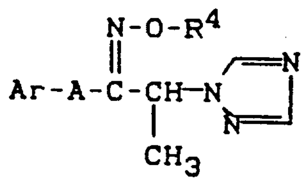

(Ig)

in welcher

Ar, A und $R^4$ die oben angegebene Bedeutung haben,

substituierte Triazole der Formel

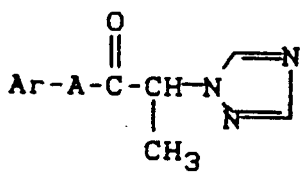

(If)

in welcher

Ar und A die oben angegebene Bedeutung haben,

mit Hydroxylamin-Derivaten der Formel

$H_2N-O-R^4$ (V)

in welcher

$R^4$ die oben angegebene Bedeutung hat,

oder mit deren Säureadditionssalzen gegebenenfalls in Gegenwart eines Verdünnungsmittels sowie gegebenenfalls in Gegenwart eines Reaktionshilfsmittels umsetzt;

(g) zur Herstellung von substituierten Triazolen der Formel

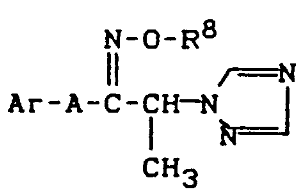

(Ih)

in welcher

Ar und A die oben angegebene Bedeutung haben und

$R^8$ für geradkettiges oder verzweigtes Alkyl mit 1 bis 6 Kohlenstoffatomen oder für Cycloalkyl mit 3 bis 7 Kohlenstoffatomen steht, wobei jeder der Cycloalkylreste einfach oder mehrfach, gleichartig oder verschieden substituiert sein kann durch

Halogen, geradkettiges oder verzweigtes Alkyl mit 1 bis 4 Kohlenstoffatomen und/oder geradkettiges oder
verzweigtes Halogenalkyl mit 1 bis 4 Kohlenstoffatomen und 1 bis 9 gleichen oder verschiedenen Halogenatomen, oder für Aralkyl mit 6 bis 10 Kohlenstoffatomen im Arylteil und 1 bis 4 Kohlenstoffatomen im geradkettigen oder verzweigten Alkylteil steht, wobei jeder der Arylreste einfach oder mehrfach, gleichartig oder verschieden substituiert sein kann durch
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy und/oder Halogenalkylthio mit jeweisl 1 bis 4 Kohlenstoffatomen und 1 bis 9 gleichen oder verschiedenen Halogenatomen,

substituierte Triazole der Formel

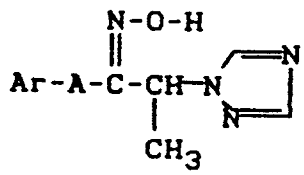

(Ii)

in welcher
Ar und A die oben angegebene Bedeutung haben,
mit Verbindungen der Formel

$R^8$-$E^1$ (VI)

in welcher
$R^8$ die oben angegebene Bedeutung hat und
$E^1$ für Chlor, Brom, Iod, Methylsulfonyloxy oder p-Methyl-phenyl-sulfonyloxy steht,
gegebenenfalls in Gegenwart eines Verdünnungsmittels und gegebenenfalls in Gegenwart eines Reaktionshilfsmittels umsetzt;
(h) zur Herstellung von substituierten Triazolen der Formel

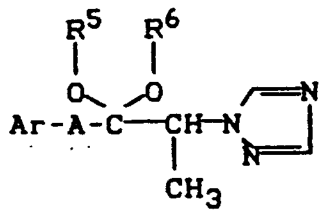

(Ij)

in welcher
Ar, A, $R^5$ und $R^6$ die oben angegebene Bedeutung haben,
substituierte Triazole der Formel

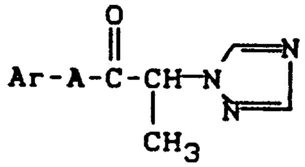

(If)

in welcher
Ar und A die oben angegebene Bedeutung haben,

α) entweder mit Alkoholen der Formeln

$R^5$ - OH (VII) oder $R^6$ - OH (VIII)

in welchen
$R^5$ und $R^6$ die oben angegebenen Bedeutungen haben,
oder
β) mit Diolen der Formel

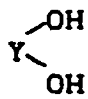

(IX)

in welcher
Y für einen 1,2-Ethandiyl-Rest steht, der einfach bis vierfach, gleichartig oder verschieden substituiert sein kann durch Methyl, Ethyl, n- oder i-Propyl, n-Butyl, Chlormethyl, Trifluormethyl, Methoxymethyl, Ethoxymethyl, Methoxyethyl, Ethoxyethyl und/oder gegebenenfalls im Phenylteil ein- bis dreifach, gleich oder verschieden durch Fluor, Chlor, Brom, Methyl und/oder Ethyl substituiertes Benzyloxymethyl,
gegebenenfalls in Gegenwart eines Verdünnungsmittels und gegebenenfalls in Gegenwart eines Reaktionshilfsmittels umsetzt,
und gegebenenfalls anschließend an die so erhaltenen substituierten Triazole der Formel (I) eine Säure oder ein Metallsalz addiert.

**3.** Fungizide Mittel, gekennzeichnet durch einen Gehalt an mindestens einem substituierten Triazol der Formel (I) gemäß Anspruch I bzw. an einem Säureadditions-Salz oder Metallsalz-Komplex eines substituierten Triazols der Formel (I).

**4.** Verwendung von substituierten Triazolen der Formel (I) gemäß Anspruch 1 bzw. von deren Säureadditions-Salzen und Metallsalz-Komplexen zur Bekämpfung von Pilzen.

**5.** Verfahren zur Bekämpfung von Pilzen, dadurch gekennzeichnet, daß man substituierte Triazole der Formel (I) gemäß Anspruch 1 bzw. deren Säureadditions-Salze oder Metallsalz-Komplexe auf die Pflanzen und/oder ihren Lebensraum ausbringt.

**6.** Verfahren zur Herstellung von fungiziden Mitteln, dadurch gekennzeichnet, daß man substituierte Triazole der Formel (I) gemäß Anspruch 1 bzw. deren Säureadditions-Salze oder Metallsalz-Komplexe mit Streckmitteln und/oder oberflächenaktiven Stoffen vermischt.

## Claims

**1.** Substituted triazoles of the formula

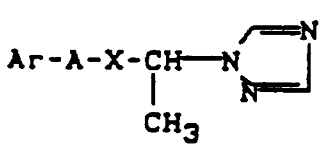

(I)

in which
Ar represents aryl which has 6 to 10 carbon atoms and which can be monosubstituted or polysubstituted by identical or different substituents from the series comprising halogen, cyano, nitro, in each case straight-chain or branched alkyl, alkoxy and alkylthio, each having 1 to 4 carbon atoms, in each case straight-chain or branched halogenoalkyl, halogenoalkoxy and halogenoalkylthio, each having 1 to 4 carbon atoms and 1 to 9 identical or different halogen atoms, in each case straight-chain or branched alkoxycarbonyl and alkoximinoalkyl, each having 1 to 4 carbon atoms in the alkoxy moiety, and 1 to 4

carbon atoms in the alkyl moiety, phenyl which is optionally monosubstituted or polysubstituted by identical or different substituents from the series comprising halogen and/or straight-chain or branched alkyl having 1 to 4 carbon atoms, phenoxy which is optionally monosubstituted or polysubstituted by identical or different substituents from the series comprising halogen and/or straight-chain or branched alkyl having 1 to 4 carbon atoms and/or by benzyloxy which is optionally monosubstituted or polysubstituted by identical or different substituents from the series comprising halogen and/or straight-chain or branched alkyl having 1 to 4 carbon atoms

A represents the group -CH=C- or

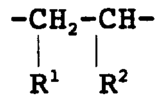

and

X represents the groups

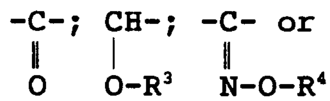

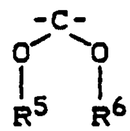

where

$R^1$ represents hydrogen or straight-chain or branched alkyl having 1 to 4 carbon atoms,

$R^2$ represents hydrogen or straight-chain or branched alkyl having 1 to 4 carbon atoms,

$R^3$ represents hydrogen, in each case straight-chain or branched alkyl having 1 to 8 carbon atoms, alkenyl having 3 to 8 carbon atoms or alkanoyl having 1 to 6 carbon atoms in the alkane moiety, furthermore represents cycloalkyl which has 3 to 7 carbon atoms and which can be monosubstituted or polysubstituted by identical or different substituents from the series comprising halogen, straight-chain or branched alkyl having 1 to 4 carbon atoms and/or straight-chain or branched halogenoalkyl having 1 to 4 carbon atoms and 1 to 9 identical or different halogen atoms, or represents aralkyl having 6 to 10 carbon atoms in the aryl moiety and 1 to 4 carbon atoms in the straight-chain or branched alkyl moiety, it being possible for each of the radicals to be monosubstituted or polysubstituted in the aryl moiety by identical or different substituents from the series comprising halogen, cyano, nitro, in each case straight-chain or branched alkyl, alkoxy or alkylthio, each having 1 to 4 carbon atoms and/or in each case straight-chain or branched halogenoalkyl, halogenoalkoxy or halogenoalkylthio, each having 1 to 4 carbon atoms and 1 to 9 identical or different halogen atoms, or represents aroyl having 6 to 10 carbon atoms in the aryl moiety, it being possible for each of these aryl radicals to be monosubstituted or polysubstituted by identical or different substituents from the series comprising halogen, cyano, nitro, in each case straight-chain or branched alkyl, alkoxy or alkylthio, each having 1 to 4 carbon atoms and/or in each case straight-chain or branched halogenoalkyl, halogenoalkoxy or halogenoalkylthio, each having 1 to 4 carbon atoms and 1 to 9 identical or different halogen atoms,

$R^4$ represents hydrogen, straight-chain or branched alkyl having 1 to 6 carbon atoms or represents cycloalkyl having 3 to 7 carbon atoms, it being possible for each of the cycloalkyl radicals to be monosubstituted or polysubstituted by identical or different substituents from the series comprising halogen, straight-chain or branched alkyl having 1 to 4 carbon atoms and/or straight-chain or branched halogenoalkyl having 1 to 4 carbon atoms and 1 to 9 identical or different halogen atoms, or represents aralkyl having 6 to 10 carbon atoms in the aryl moiety and 1 to 4 carbon atoms in the straight-chain or branched alkyl moiety, it being possible for each of the alkyl radicals to be monosubstituted or polysubstituted by identical or different substituents from the series comprising halogen, cyano, nitro, in each case straight-chain or branched alkyl, alkoxy or alkylthio, each having 1 to 4 carbon atoms, in

each case straight-chain or branched halogenoalkyl, halogenoalkoxy and/or halogenoalkylthio, each having 1 to 4 carbon atoms and 1 to 9 identical or different halogen atoms, or represents aryl having 6 to 10 carbon atoms, it being possible for each of the aryl radicals to monosubstituted or polysubstituted by identical or different substituents from the series comprising halogen, cyano, nitro, in each case straight-chain or branched alkyl, alkoxy or alkylthio, each having 1 to 4 carbon atoms, in each case straight-chain or branched halogenoalkyl, halogenoalkoxy and/or halogenoalkylthio, each having 1 to 4 carbon atoms and 1 to 9 identical or different halogen atoms and

$R^5$ and $R^6$ independently of one another represent alkyl having 1 to 6 carbon atoms or aralkyl having 6 to 10 carbon atoms in the aryl moiety and 1 to 4 carbon atoms in the straight-chain or branched alkyl moiety, it being possible for each of the aryl radicals to be monosubstituted or polysubstituted by identical or different substituents from the series comprising halogen, cyano, nitro, in each case straight-chain or branched alkyl, alkoxy or alkylthio, each having 1 to 4 carbon atoms, in each case straight-chain or branched halogenoalkyl, halogenoalkoxy and/or halogenoalkylthio, each having 1 to 4 carbon atoms and 1 to 9 identical or different halogen atoms,

or

$R^5$ and $R^6$ together represent an alkylene radical having 2 to 4 carbon atoms, it being possible for the alkylene radical to be monosubstituted or polysubstituted by identical or different substituents from the series comprising straight-chain or branched alkyl having 1 to 4 carbon atoms, straight-chain or branched halogenoalkyl having 1 to 4 carbon atoms and 1 to 9 identical or different halogen atoms, alkoxyalkyl having 1 to 4 carbon atoms in the alkyl moiety and 1 to 4 carbon atoms in the alkoxy moiety and/or aralkyloxyalkyl 6 to 10 carbon atoms in the aryl moiety and 1 to 4 carbon atoms in the straight-chain or branched alkoxy moiety and also 1 to 4 carbon atoms in the straight-chain or branched alkyl moiety, it being possible for the aryl radical to be monosubstituted or polysubstituted by identical or different substituents from the series comprising halogen and/or alkyl having 1 to 4 carbon atoms and their acid addition salts and metal salt complexes.

**2.** Processes for the preparation of substituted triazoles of the formula (I) according to Claim 1 and their acid addition salts and metal salt complexes, characterized in that

(a) for the preparation of substituted triazoles of the formula

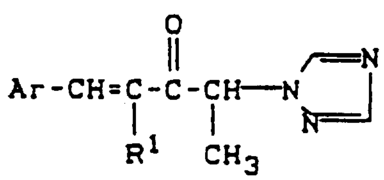

(Ia)

in which

Ar and $R^1$ have the abovementioned meaning, aromatic aldehydes of the formula

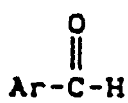

(II)

in which

Ar has the abovementioned meaning,

are reacted with triazolylketones of the formula

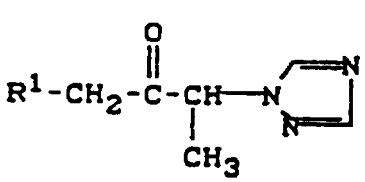

(III)

in which
$R^1$ has the abovementioned meaning,
if appropriate in the presence of a diluent and if appropriate in the presence of a reaction auxiliary;
(b) for the preparation of substituted triazoles of the formula

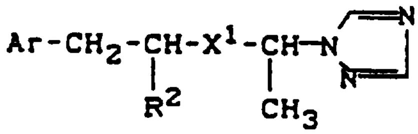

(Ib)

in which
$X^1$ represents one of the groups

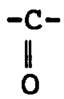

or

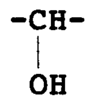

and
Ar and $R^2$ have the abovementioned meaning,
substituted triazoles of the formula

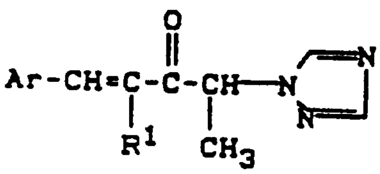

(Ia)

in which
Ar and $R^1$ have the abovementioned meaning
are hydrogenated with hydrogen in the presence of a hydrogenation catalyst and in the presence of a diluent;
(c) for the preparation of substituted triazoles of the formula

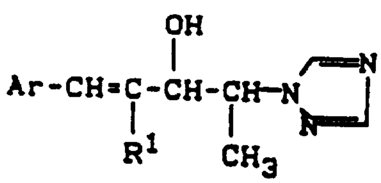

(Ic)

in which
Ar and $R^1$ have the abovementioned meaning,
substituted triazoles of the formula

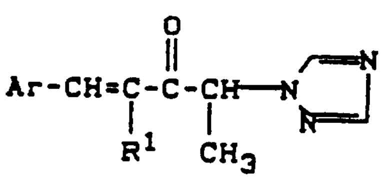

(Ia)

in which
Ar and $R^1$ have the abovementioned meaning,

are reduced with complex hydrides in the presence of a diluent;
(d) for the preparation of substituted triazoles of the formula

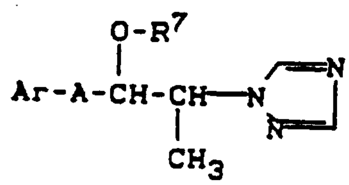

(Id)

in which
$R^7$ represents in each case straight-chain or branched alkyl having 1 to 8 carbon atoms, alkenyl having 3 to 8 carbon atoms or alkanoyl having 1 to 6 carbon atoms in the alkane moiety, furthermore represents cycloalkyl which has 3 to 7 carbon atoms and which can be monosubstituted or polysubstituted by identical or different substituents from the series comprising halogen, straight-chain or branched alkyl having 1 to 4 carbon atoms and/or straight-chain or branched halogenoalkyl having 1 to 4 carbon atoms and 1 to 9 identical or different halogen atoms, or represents aralkyl having 6 to 10 carbon atoms in the aryl moiety and 1 to 4 carbon atoms in the straight-chain or branched alkyl moiety, it being possible for each of the radicals to be monosubstituted or polysubstituted in the aryl moiety by identical or different substituents from the series comprising halogen, cyano, nitro, in each case straight-chain or branched alkyl, alkoxy or alkylthio, each having 1 to 4 carbon atoms and/or in each case straight-chain or branched halogenoalkyl, halogenoalkoxy or halogenoalkylthio, each having 1 to 4 carbon atoms and 1 to 9 identical or different halogen atoms, or represents aroyl having 6 to 10 carbon atoms in the aryl moiety, it being possible for each of these aryl radicals to be monosubstituted or polysubstituted by identical or different substituents from the series comprising halogen, cyano, nitro, in each case straight-chain or branched alkyl, alkoxy or alkylthio, each having 1 to 4 carbon atoms and/or in each case straight-chain or branched halogenoalkyl, halogenoalkoxy or halogenoalkylthio, each having 1 to 4 carbon atoms and 1 to 9 identical or different halogen atoms,
Ar and A have the abovementioned meaning, substituted triazoles of the formula

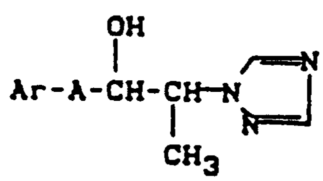

(Ie)

in which
Ar and A have the abovementioned meaning, are reacted with compounds of the formula

$R^7$-E (IV)

in which
$R^7$ has the abovementioned meaning and
E represents chlorine, bromine, iodine, methylsulphonyloxy, p-methyl-phenyl-sulphonyloxy or an acetic anhydride or propionic anhydride radical,
if appropriate in the presence of a diluent and if appropriate in the presence of a reaction auxiliary;
(e) for the preparation of substituted triazoles of the formula

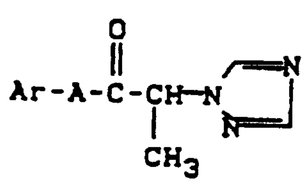

(If)

in which

Ar and A have the abovementioned meaning,
substituted triazoles of the formula

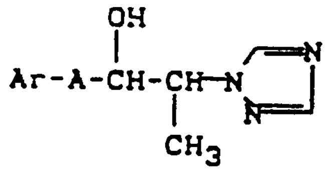

(Ie)

in which
Ar and A have the abovementioned meaning
are reacted with an oxidating agent if appropriate in the presence of a diluent;
(f) for the preparation of substituted triazoles of the formula

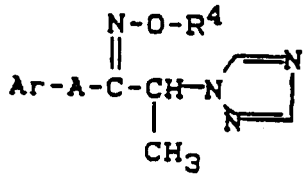

(Ig)

in which
Ar, A and $R^4$ have the abovementioned meaning,
substituted triazoles of the formula

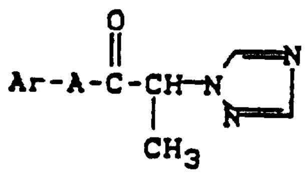

(If)

in which
Ar and A have the abovementioned meaning,
are reacted with hydroxylamine derivatives of the formula

$H_2N\text{-}O\text{-}R^4$ (V)

in which
$R^4$ has the abovementioned meaning
or with their acid addition salts if appropriate in the presence of a diluent and if appropriate in the presence of a reaction auxiliary;
(g) for the preparation of substituted triazoles of the formula

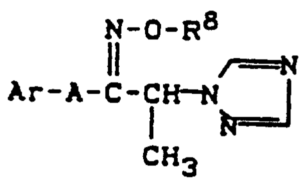

(Ih)

in which
Ar and A have the abovementioned meaning and
$R^8$ represents straight-chain or branched alkyl having 1 to 6 carbon atoms or represents cycloalkyl having 3 to 7 carbon atoms, it being possible for each of the cycloalkyl radicals to be monosubstituted or polysubstituted by identical or different substituents from the series comprising halogen, straight-chain or branched alkyl having 1 to 4 carbon atoms and/or straight-chain or branched halogenoalkyl having 1 to 4 carbon atoms and 1 to 9 identical or different halogen atoms, or represents aralkyl having 6 to 10 carbon atoms in the aryl moiety and 1 to 4 carbon atoms in the straight-chain or branched alkyl moiety, it being possible for each of the alkyl radicals to be monosubstituted or polysubstituted by identical or different substituents from the series comprising halogen, cyano, nitro, in each case straight-chain or branched alkyl, alkoxy or alkylthio, each having 1 to 4 carbon atoms, in each case straight-chain or branched halogenoalkyl, halogenoalkoxy and/or halogenoalkylthio, each having 1 to 4 carbon atoms and 1 to 9 identical or different halogen atoms, substituted triazoles of the formula

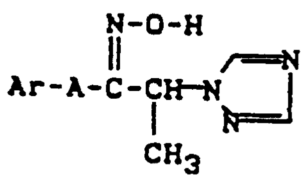 (Ii)

in which
Ar and A have the abovementioned meaning,
are reacted with compounds of the formula

$R^8$-$E^1$ (VI)

in which
$R^8$ has the abovementioned meaning and
$E^1$ represents chlorine, bromine, iodine, methylsulphonyloxy or p-methyl-phenyl-sulphonyloxy,
if appropriate in the presence of a diluent and if appropriate in the presence of an reaction auxiliary;
(h) for the preparation of substituted triazoles of the formula

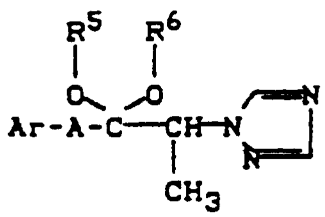 (Ij)

in which
Ar, A, $R^5$ and $R^6$ have the abovementioned meaning,
substituted triazoles of the formula

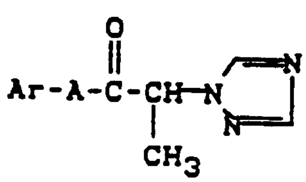 (If)

in which
Ar and A have the abovementioned meaning
are reacted either
α) with alcohols of the formulae

$R^5$-OH (VII) or $R^6$-OH (VIII)

in which
$R^5$ and $R^6$ have the abovementioned meanings
or
$\beta$) with diols of the formula

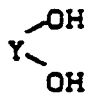

(IX)

in which
Y represents a 1,2-ethanediyl radical which can be monosubstituted to tetrasubstituted by identical or different substituents selected from the series comprising methyl, ethyl, n- or i-propyl, n-butyl, chloromethyl, trifluoromethyl, methoxymethyl, ethoxymethyl, methoxyethyl, ethoxyethyl and/or benzyloxymethyl which is optionally mono to trisubstituted in the phenyl moriety by identical or different substituents from the series comprising fluorine, chlorine, bromine, methyl and/or ethyl,
if appropriate in the presence of a diluent and if appropriate in the presence of a reaction auxiliary, and when, if desired, an acid or a metal salt is subjected to an addition reaction with the resulting substituted triazoles of the formula (I).

**3.** Fungicidal agents, characterized in that they contain at least one substituted triazole of the formula (I) according to Claim 1 or an acid addition salt or metal salt complex of a substituted triazole of the formula (I).

**4.** Use of substituted triazoles of the formula (I) according to Claim 1 or of their acid addition salts and metal salt complexes for combating fungi.

**5.** Method of combating fungi, characterized in that substituted triazoles of the formula (I) according to Claim 1 or their acid addition salts or metal salt complexes are applied to the plants and/or their environment.

**6.** Process for the preparation of fungicidal agents, characterized in that substituted triazoles of the formula (I) according to Claim 1 or their acid addition salts or metal salt complexes are mixed with extenders and/or surface-active substances.

## Revendications

**1.** Triazoles substitués de formule :

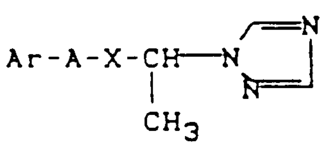

(I)

dans laquelle

Ar représente un groupe aryle ayant 6 à 10 atomes de carbone, qui peut être substitué une ou plusieurs fois, de façon identique ou différente, par de l'halogène, par un reste cyano, nitro, par un reste alkyle, alcoxy ou alkylthio chacun linéaire ou ramifié et ayant chacun 1 à 4 atomes de carbone, un reste halogénoalkyle, halogénoalcoxy, et halogénoalkylthio, chacun linéaire ou ramifié ayant chacun 1 à 4 atomes de carbone et 1 à 9 atomes d'halogène, identiques ou différents, un groupe alcoxy carbonyle et alcoxyiminoalkyle chacun linéaire ou ramifié et ayant chacun 1 à 4 atomes de carbone dans la partie alcoxy et 1 à 4 atomes de carbone dans la partie alkyle, un groupe phényle éventuellement substitué une ou plusieurs fois, de façon identique ou différente, par de l'halogène et/ou par un reste alkyle linéaire ou ramifié ayant 1 à 4 atomes de carbone, un groupe phénoxy éventuellement substitué une ou

plusieurs fois, de façon identique ou différente, par de l'halogène et/ou par un reste alkyle linéaire ou ramifié ayant 1 à 4 atomes de carbone et/ou un groupe benzyloxy éventuellement substitué une ou plusieurs fois de façon identique ou différente par de l'halogène et/ou par un reste alkyle linéaire ou ramifié ayant 1 à 4 atomes de carbone,

A représente le groupe

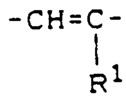 ou 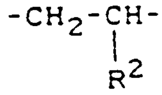

et

X représente le groupe

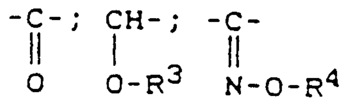 ou 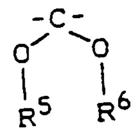

formules dans lesquelles

$R^1$ représente un atome d'hydrogène ou un groupe alkyle linéaire ou ramifié ayant 1 à 4 atomes de carbone,

$R^2$ représente un atome d'hydrogène ou un groupe alkyle linéaire ou ramifié ayant 1 à 4 atomes de carbone,

$R^3$ représente un atome d'hydrogène, un groupe alkyle ayant 1 à 8 atomes de carbone, alcényle ayant 2 à 8 atomes de carbone, alcanoyle ayant 1 à 6 atomes de carbone dans la partie alcane, chacun linéaire ou ramifié, et en outre $R^3$ représente un groupe cycloalkyle ayant 3 à 7 atomes de carbone, qui peut être substitué une ou plusieurs fois, de façon identique ou différente par de l'halogène, par un reste alkyle linéaire ou ramifié ayant 1 à 4 atomes de carbone et/ou un reste halogénoalkyle linéaire ou ramifié ayant 1 à 4 atomes de carbone et 1 à 9 atomes d'halogène identiques ou différents, ou bien $R^3$ représente un groupe aralkyle ayant 6 à 10 atomes de carbone dans la partie aryle et 1 à 4 atomes de carbone dans la partie alkyle linéaire ou ramifiée, chacun des restes pouvant être substitué une ou plusieurs fois, de façon identique ou différente dans la partie aryle par de l'halogène, par un reste cyano, nitro, un reste alkyle, alcoxy ou alkylthio linéaire ou ramifié ayant chacun 1 à 4 atomes de carbone et/ou par un reste halogénoalkyle, halogénoalcoxy ou halogénoalkylthio, chacun linéaire ou ramifié et ayant chacun 1 à 4 atomes de carbone et 1 à 9 atomes d'halogène identiques ou différents, ou $R^3$ représente un groupe aroyle ayant 6 à 10 atomes de carbone dans la partie aryle, chacun de ces restes aryle pouvant être substitué une ou plusieurs fois, de façon identique ou différente par de l'halogène, par un reste cyano, nitro, par un reste alkyle, alcoxy ou alkylthio chacun linéaire ou différent et comportant chacun 1 à 4 atomes de carbone et/ou par un reste halogénoalkyle, halogénoalcoxy ou halogénoalkylthio, chacun linéaire ou ramifié et comportant chacun 1 à 4 atomes de carbone et 1 à 9 atomes d'halogène identiques ou différents,

$R^4$ représente un atome d'hydrogène, un groupe alkyle linéaire ou ramifié ayant 1 à 6 atomes de carbone ou un groupe cycloalkyle ayant 3 à 7 atomes de carbone, chacun des restes cycloalkyle pouvant être substitué une ou plusieurs fois, de façon identique ou différente, par de l'halogène, par un reste alkyle linéaire ou ramifié ayant 1 à 4 atomes de carbone et/ou par un reste halogénoalkyle linéaire ou ramifié ayant 1 à 4 atomes de carbone et 1 à 9 atomes d'halogène identiques ou différents, ou bien $R^4$ représente un groupe aralkyle ayant 6 à 10 atomes de carbone dans la partie aryle et 1 à 4 atomes de carbone dans la partie alkyle linéaire ou ramifiée, chacun des restes aryle pouvant être substitué une ou plusieurs fois, de façon identique ou différente, par de l'halogène, par un reste cyano, nitro, par un reste alkyle, alcoxy, ou alkylthio, chacun linéaire ou ramifié et ayant chacun 1 à 4 atomes de carbone, par un reste halogénoalkyle, halogénoalcoxy et/ou halogénoalkylthio, chacun linéaire ou ramifié et ayant chacun 1 à 4 atomes de carbone et 1 à 9 atomes d'halogène identiques ou différents, ou bien $R^4$ représente un groupe aryle ayant 6 à 10 atomes de carbone, chacun des restes aryle pouvant être substitué une ou plusieurs fois, de façon identique ou différente par de l'halogène, par un reste cyano, nitro, par un reste alkyle, alcoxy ou alkylthio, chacun linéaire ou ramifié et ayant chacun 1 à 4 atomes de carbone, par un reste halogénoalkyle, halogénoalcoxy et/ou halogénoalkylthio, chacun linéaire ou ramifié et ayant chacun 1 à 4 atomes de carbone et 1 à 9 atomes d'halogène identiques ou différents.

$R^5$ et $R^6$ représentent chacun, indépendamment l'un de l'autre, un groupe alkyle ayant 1 à 4 atomes de carbone ou aralkyle ayant 6 à 10 atomes de carbone dans la partie aryle et 1 à 4 atomes de carbone dans la partie alkyle linéaire ou ramifiée, chacun des restes aryles pouvant être substitué une ou plusieurs fois, de façon identique ou différente, par de l'halogène, par un reste cyano, nitro, par un reste alkyle, alcoxy et alkylthio chacun linéaire ou ramifié et comportant chacun 1 à 4 atomes de carbone, par un reste halogénoalkyle, halogénoalcoxy et/ou halogénoalkylthio, chacun linéaire ou ramifié et comportant chacun 1 à 4 atomes de carbone et 1 à 9 atomes d'halogène identiques ou différents, ou

$R^5$ et $R^6$ forment ensemble un reste alkylène comportant 2 à 4 atomes de carbone, le reste alkylène pouvant être substitué une ou plusieurs fois, de façon identique ou différente, par un reste alkyle linéaire ou ramifié ayant 1 à 4 atomes de carbone, par un reste halogénoalkyle linéaire ou ramifié ayant 1 à 4 atomes de carbone et 1 à 9 atomes d'halogène identiques ou différents, par un reste alcoxyalkyle ayant 1 à 4 atomes de carbone dans la partie alkyle et 1 à 4 atomes de carbone dans la partie alcoxy et/ou par un reste arylalkyloxyalkyle comportant 6 à 10 atomes de carbone dans la partie aryle et 1 à 4 atomes de carbone dans la partie alkyloxy linéaire ou ramifiée ainsi que 1 à 4 atomes de carbone dans la partie alkyle linéaire ou ramifiée, le reste aryle pouvant être substitué une ou plusieurs fois, de façon identique ou différente, par de l'halogène et/ou par un reste alkyle ayant 1 à 4 atomes de carbone,

ainsi que leurs sels d'addition d'acide et leurs complexes avec des sels de métaux.

**2.** Procédé pour préparer des triazoles substitués de formule (I) selon la revendication 1, ainsi que leurs sels d'addition d'acides et leurs complexes avec des sels de métaux, procédé caractérisé en ce que

(a) pour préparer des triazoles substitués de formule :

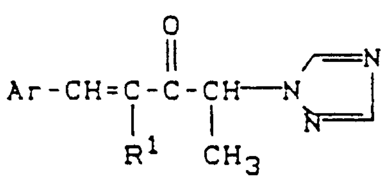

(Ia)

dans laquelle

Ar et $R^1$ ont le sens indiqué ci-dessus,

on fait réagir des aldéhydes aromatiques de formule :

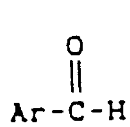

(II)

dans laquelle

Ar a le sens indiqué ci-dessus,

avec des triazolylcétones de formule :

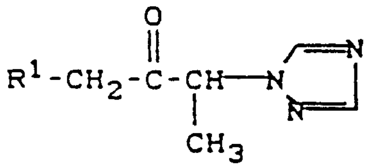

(III)

dans laquelle

$R^1$ a le sens indiqué ci-dessus,

en opérant éventuellement en présence d'un diluant et éventuellement en présence d'un adjuvant facilitant la réaction ;

(b) pour préparer des triazoles substitués de formule :

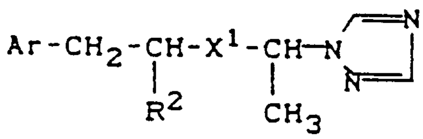

(Ib)

dans laquelle

$X^1$ représente un des groupes

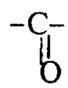 ou 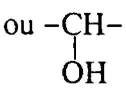

et

Ar et $R^2$ ont le sens indiqué ci-dessus,

on soumet des triazoles substitués de formule :

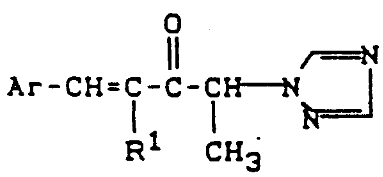

(Ia)

dans laquelle

Ar et R ¹ ont le sens indiqué ci-dessus,

à une hydrogénation effectuée avec de l'hydrogène en présence d'un catalyseur d'hydrogénation et en présence d'un diluant ;

(c) pour préparer des triazoles substitués de formule :

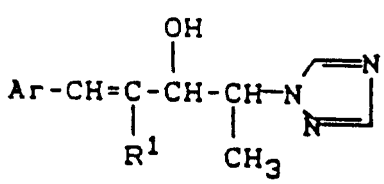

(Ic)

dans laquelle

Ar et $R^1$ ont le sens indiqué ci-dessus,

on soumet des triazoles substiutés, de formule :

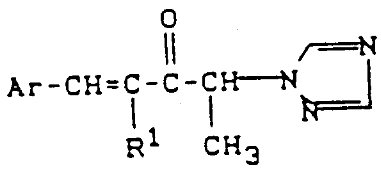

(Ia)

dans laquelle

Ar et $R^1$ ont le sens indiqué ci-dessus,

à une réduction effectuée à l'aide d'hydrures complexes en présence d'un diluant,

(d) pour préparer des triazoles substitués de formule :

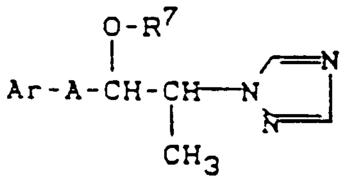

(Id)

dans laquelle

$R^7$ représente un groupe alkyle ayant 1 à 8 atomes de carbone, alcényle ayant 3 à 8 atomes de carbone ou alcanoyle ayant 1 à 6 atomes de carbone dans la partie alcane, chacun étant linéaire ou ramifié, $R^7$ pouvant représenter en outre un groupe cycloalkyle ayant 3 à 7 atomes de carbone, qui peut être substitué une ou plusieurs fois, de façon identique ou différente par de l'halogène, par un groupe alkyle linéaire ou ramifié ayant 1 à 4 atomes de carbone et/ou par un groupe halogénoalkyle linéaire ou ramifié ayant 1 à 4 atomes de carbone et 1 à 9 atomes d'halogène identiques ou différents, ou un groupe aralkyle ayant 1 à 4 atomes de carbone dans la partie alkyle linéaire ou ramifiée, chacun des restes pouvant être substitué une ou plusieurs fois de façon identique ou différente dans la partie aryle, par de l'halogène, par un reste cyano, nitro, par un reste alkyle, alcoxy ou alkylthio chacun linéaire ou ramifié et comportant chacun 1 à 4 atomes de carbone et/ou par un reste halogénoalkyle, halogénoalcoxy ou halogénoalkylthio, chacun linéaire ou ramifié et comportant 1 à 9 atomes d'halogène identiques ou différents, ou bien $R^7$ représente un groupe aroyle ayant 6 à 10 atomes de carbone dans la partie aryle, chacun de ces restes aryle pouvant être substitué une ou plusieurs fois, de façon identique ou différente par de l'halogène, par un reste cyano, nitro, par un reste alkyle, alcoxy ou alkylthio chacun linéaire ou ramifié et comportant chacun 1 à 4 atomes de carbone et/ou par un reste halogénoalkyle, halogénoalcoxy, ou halogénoalkylthio, chacun linéaire ou ramifié et comportant chacun 1 à 4 atomes de carbone et 1 à 9 atomes d'halogène identiques ou différents,

Ar et A ont le sens indiqué ci-dessus,

on fait réagir éventuellement en présence d'un diluant et éventuellement en présence d'un agent facilitant la réaction, des triazoles substitués de formule :

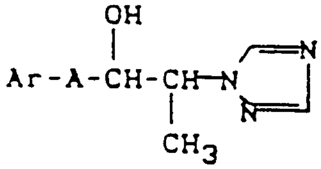

(Ie)

dans laquelle

Ar et A ont le sens indiqué ci-dessus,

avec des composés de formule :

$R^7$-E (IV)

dans laquelle

$R^7$ a le sens indiqué ci-dessus et

E représente un atome de chlore, de brome, d'iode, un reste méthylsulfonyloxy, p-méthyl-phényl-sulfonyloxy ou un reste anhydride d'acide acétique ou d'acide propionique,

(e) pour préparer des triazoles substitués de formule :

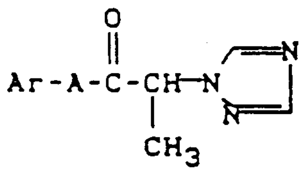

(If)

dans laquelle

Ar et A ont le sens indiqué ci-dessus,

on fait réagir des triazoles substitués de formule :

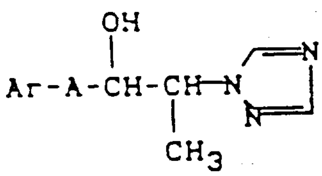

(Ie)

dans laquelle

Ar et A ont le sens indiqué ci-dessus,

avec un oxydant, éventuellement en présence d'un diluant;

(f) pour préparer des triazoles substitués de formule :

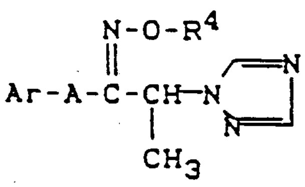

(Ig)

dans laquelle

Ar, A et $R^4$ ont le sens indiqué ci-dessus,

on fait réagir des triazoles substitués de formule :

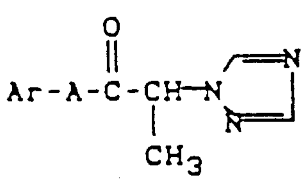

(If)

dans laquelle

Ar et A ont le sens indiqué ci-dessus, avec des dérivés de l'hydroxylamine, de formule :

$H_2N$-O-$R^4$ (V)

dans laquelle

$R^4$ a le sens indiqué ci-dessus,

ou avec leurs sels d'addition d'acide, en opérant éventuellement en présence d'un diluant et éventuellement en présence d'un adjuvant facilitant la réaction ;

(g) pour préparer des triazoles substitués de formule :

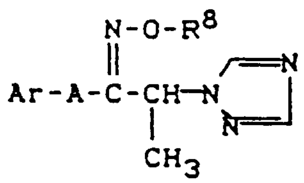 (Ih)

dans laquelle

Ar et A ont le sens indiqué ci-dessus,

et

$R^8$ représente un groupe alkyle linéaire ou ramifié ayant 1 à 6 atomes de carbone ou un groupe cycloalkle ayant 3 à 7 atomes de carbone, chacun des restes cycloalkyle pouvant être substitué une ou plusieurs fois, de façon identique ou différente, par de l'halogène, par un reste alkyle linéaire ou ramifié ayant 1 à 4 atomes de carbone et/ou par un reste halogénoalkyle linéaire ou ramifié ayant 1 à 4 atomes de carbone et 1 à 9 atomes d'halogène identiques ou différents, ou bien $R^8$ représente un groupe aralkyle comportant 6 à 10 atomes de carbone dans la partie aryle et 1 à 4 atomes de carbone dans la partie alkyle linéaire ou ramifiée, chacun des restes aryle pouvant être substitué une ou plusieurs fois, de façon identique ou différente, par de l'halogène, par un reste cyano, nitro, par un reste alkyle, alcoxy ou alkylthio chacun linéaire ou ramifié et comportant chacun 1 à 4 atomes de carbone, par un reste halogénoalkyle, halogénoalcoxy et/ou halogénoalkylthio, comportant chacun 1 à 4 atomes de carbone et 1 à 9 atomes d'halogène identiques ou différents,

on fait réagir, éventuellement en présence d'un diluant et éventuellement en présence d'un adjuvant facilitant la réaction, des triazoles substitués de formule :

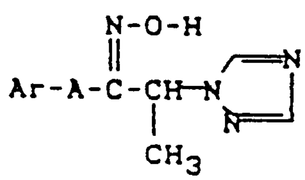 (Ii)

dans laquelle

Ar et A ont le sens indiqué ci-dessus,

avec des composés de formule :

$R^8$-$E^1$ (VI)

dans lesquelles

$R^8$ a le sens indiqué ci-dessus, et

$E^1$ représente un atome de chlore, de brome, d'iode ou un groupe méthylsulfonyloxy ou p-méthyl-phényl-sulfonyloxy ;

(h) pour préparer des triazoles substiutés de formule :

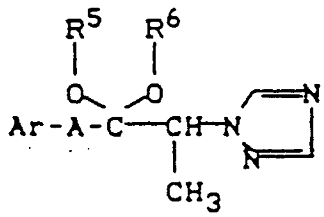

(Ij)

dans laquelle

Ar, A, $R^5$ et $R^6$ ont le sens indiqué ci-dessus,

on fait réagir, éventuellement en présence d'un diluant et éventuellement en présence d'un adjuvant facilitant la réaction,

des triazoles substitués de formule :

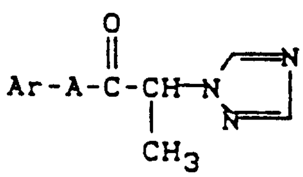

(If)

dans laquelle

Ar et A ont le sens indiqué ci-dessus,

$\alpha$) avec des alcools de formule :

$R^5$ - OH (VII) ou $R^6$ - OH (VIII)

dans lesquelles

$R^5$ et $R^6$ ont les sens indiqués ci-dessus,

ou

$\beta$) avec des diols de formule :

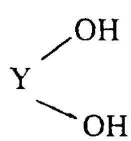

(IX)

dans laquelle

Y représente un reste 1,2-éthanediyle, qui peut être substitué une ou plusieurs fois, de façon identique ou différente, par un reste méthyle, éthyle, n- ou i-propyle, n-butyle, chlorométhyle, trifluorométhyle, méthoxyméthyle, éthoxyméthyle, méthoxyéthyle, éthoxyéthyle et/ou par un reste benzyloxyméthyle éventuellement substitué dans la partie phényle 1 à 3 fois, de façon identique ou différente, par un atome de fluor, de chlore ou de brome ou par un groupe méthyle et/ou éthyle,

et éventuellement en fixant ensuite par addition, sur les triazoles substitués de formule (I) que l'on obtient ainsi, un acide ou un sel de métal.

**3.** Produits fongicides, caractérisés en ce qu'ils contiennent au moins un triazole substitué de formule (I) selon la revendication 1 ou un sel d'addition d'acide ou un complexe avec un sel de métal d'un triazole substitué de formule (I).

4. Utilisation de triazoles substitués de formule (I) selon la revendication 1, ou de leurs sels d'addition d'acide ou de leurs complexes avec un sel de métal pour combattre des champignons.

5. Procédé pour combattre des champignons, caractérisé en ce qu'on applique sur les plantes et/ou sur leur espace vital ou biotope des triazoles substitués de formule (I) selon la revendication 1 ou leurs sels d'addition d'acides ou des complexes formés avec un sel de métal.

6. Procédé pour produire des produits fongicides, caractérisé en ce qu'on mélange des triazoles substitués de formule (I) selon la revendication 1, ou leurs sels d'addition d'acides ou leurs complexes formés avec un sel de métal, avec des agents d'allongement et/ou avec des substances tensioactives.